# EUROPEAN PATENT SPECIFICATION

(11) **EP 1 812 046 B1**
(45) Date of publication and mention of the grant of the patent: **16.03.2011**
(21) Application number: 05819741.9
(22) Date of filing: 25.10.2005
(51) Int. Cl.: A61K 39/395, A61K 38/34, C07K 14/68, C07K 14/725, C07K 16/46, C12N 5/10, C12N 15/11, C12N 15/16, C12N 15/62, C12N 15/86

(54) **MELANOCORTIN RECEPTOR BINDING MIMETIBODIES, COMPOSITIONS, METHODS AND USES**
MELANOCORTIN-REZEPTOR-BINDENDE MIMETIBODIES, ZUSAMMENSETZUNGEN, VERFAHREN UND VERWENDUNGEN
CORPS MIMETIQUES DE LIAISON AU RECEPTEUR DE LA MELANOCORTINE, COMPOSITIONS, PROCEDES ET UTILISATIONS

(30) Priority: 25.10.2004 US 621960 P
(43) Date of publication of application: 01.08.2007
(73) Proprietor: Centocor Ortho Biotech Inc., Horsham, PA 19044 (US)
(72) Inventor: CUNNINGHAM, Mark, Kennett Square, Pennsylvania 19348 (US); STOJANOVIC-SUSULIC, Vedrana, Princeton Junction, Pennsylvania 08550 (US); O'NEIL, Karyn, Kennett Square, Pennsylvania 19348 (US); HUANG, Chichi, Berwyn, Pennsylvania 19312 (US); LUO, Jeffrey, Malvern, Pennsylvania 19355 (US)
(74) Representative: Mercer, Christopher Paul
(86) International application number: PCT/US2005/038431
(87) International publication number: WO 2006/047535

(56) References cited:
- WO-A-02/06316
- WO-A1-00/33658
- WO-A2-2004/002424
- US-A1- 2005 191 301

### SEQUENCE LISTING

<110> Centocor, Inc.
<120> Melanocortin Receptor Binding Mimetibodies, Compositions, Methods and Uses
<130> CEN5080 PCT
<140> To Be Assigned
   <141> 2005-10-25
<150> 60/621,960
   <151> 2004-10-25
<160> 85
<170> PatentIn version 3 - 2
<210> 1
   <211> 39
   <212> DNA
   <213> Homo sapiens
<400> 1
   tcctactcca tggagcactt ccgctggggc aagccggtg 39
<210> 2
   <211> 13
   <212> PRT
   <213> Homo sapiens
<400> 2
   Ser Tyr Ser Met Glu His Phe Arg Trp Gly Lys Pro Val
   1               5                   10
<210> 3
   <211> 39
   <212> DNA
   <213> Artificial Sequence
<220>
   <223> Constrained peptide
<400> 3
   agctatagct gcgaacattt tcgctggtgc aaaccggtg 39
<210> 4
   <211> 13
   <212> PRT
   <213> Artificial Sequence
<220>
   <223> Constrained peptide
<400> 4
   Ser Tyr Ser Cys Glu His Phe  Arg Trp Cys Lys Pro Val
   1               5                    10
<210> 5
   <211> 39
   <212> DNA
   <213> Artificial Sequence
<220>
   <223> Constrained peptide
<400> 5
   agctattgca tggaacattt tcgctggtgc aaaccggtg 39
<210> 6
   <211> 13
   <212> PRT
   <213> Artificial Sequence
<220>
   <223> Constrained peptide
<400> 6
   Ser Tyr Cys Met Glu His Phe  Arg Trp Cys Lys Pro Val
   1               5                    10
<210> 7
   <211> 39
   <212> DNA
   <213> Artificial Sequence
<220>
   <223> Constrained peptide
<400> 7
   agctgcagca tggaacattt tcgctggtgc aaaccggtg 39
<210> 8
   <211> 13
   <212> PRT
   <213> Artificial Sequence
<220>
   <223> Constrained peptide
<400> 8
   Ser Cys Ser Met Glu His Phe  Arg Trp Cys Lys Pro Val
   1               5                    10
<210> 9
   <211> 39
   <212> DNA
   <213> Artificial Sequence
<220>
   <223> Constrained peptide
<400> 9
   tgctatagca tggaacattt tcgctggggc tgcccggtg 39
<210> 10
   <211> 13
   <212> PRT
   <213> Artificial Sequence
<220>
   <223> Constrained peptide
<400> 10
   Cys Tyr Ser Met Glu His Phe Arg Trp Gly Cys Pro Val
   1 5 10
<210> 11
   <211> 45
   <212> DNA
   <213> Artificial Sequence
<220>
   <223> Constrained peptide
<400> 11
   agctggagct gggaacattt tcgctggggc aaatggacct ggaaa 45
<210> 12
   <211> 15
   <212> PRT
   <213> Artificial Sequence
<220>
   <223> Constrained peptide
<400> 12
   Ser Trp Ser Trp Glu His Phe Arg Trp Gly Lys Trp Thr Trp Lys
   1 5 10 15
<210> 13
   <211> 51
   <212> DNA
   <213> Artificial Sequence
<220>
   <223> Constrained peptide
<400> 13
   agctggagct ggggcgaaca ttttcgctgg ggcaaaggct ggacctggaa a 51
<210> 14
   <211> 17
   <212> PRT
   <213> Artificial Sequence
<220>
   <223> Constrained peptide
<400> 14
   Ser Trp Ser Trp Gly Glu His Phe Arg Trp Gly Lys Gly Trp Thr Trp
   1 5 10 15
   Lys
<210> 15
   <211> 57
   <212> DNA
   <213> Artificial Sequence
<220>
   <223> Constrained peptide
<400> 15
   agctggagct ggggcggcga acattttcgc tggggcaaag gcggctggac ctggaaa 57
<210> 16
   <211> 19
   <212> PRT
   <213> Artificial Sequence
<220>
   <223> Constrained peptide
<400> 16
   Ser Trp Ser Trp Gly Gly Glu His Phe Arg Trp Gly Lys Gly Gly Trp
   1 5 10 15
   Thr Trp Lys
<210> 17
   <211> 57
   <212> DNA
   <213> Artificial Sequence
<220>
   <223> Constrained peptide
<400> 17
   agctggagct ggagcatgga acattttcgc tggggcaaac cggtgtggac ctggaaa 57
<210> 18
   <211> 19
   <212> PRT
   <213> Artificial Sequence
<220>
   <223> Constrained peptide
<400> 18
   Ser Trp Ser Trp Ser Met Glu His Phe Arg Trp Gly Lys Pro Val Trp
   1 5 10 15
   Thr Trp Lys
<210> 19
   <211> 12
   <212> DNA
   <213> Artificial Sequence
<220>
   <223> Flexible peptide
<400> 19
   ggcagcggca gc 12
<210> 20
   <211> 4
   <212> PRT
   <213> Artificial Sequence
<220>
   <223> Flexible peptide
<400> 20
   Gly Ser Gly Ser
   1
<210> 21
   <211> 12
   <212> DNA
   <213> Artificial Sequence
<220>
   <223> Flexible peptide
<400> 21
   ggcggcagcg gc 12
<210> 22
   <211> 4
   <212> PRT
   <213> Artificial Sequence
<220>
   <223> Flexible peptide
<400> 22
   Gly Gly Ser Gly
   1
<210> 23
   <211> 18
   <212> DNA
   <213> Artificial Sequence
<220>
   <223> Flexible peptide
<400> 23
   ggcagcggcg gcggcagc 18
<210> 24
   <211> 6
   <212> PRT
   <213> Artificial Sequence
<220>
   <223> Flexible peptide
<400> 24
   Gly Ser Gly Gly Gly Ser
   1 5
<210> 25
   <211> 21
   <212> DNA
   <213> Artificial Sequence
<220>
   <223> Flexible peptide
<400> 25
   ggcagcggcg gcggcagcgg c 21
<210> 26
   <211> 7
   <212> PRT
   <213> Artificial Sequence
<220>
   <223> Flexible peptide
<400> 26
   Gly Ser Gly Gly Gly Ser Gly
   1 5
<210> 27
   <211> 12
   <212> DNA
   <213> Artificial Sequence
<220>
   <223> Flexible peptide
<400> 27
   ggcagcagcg gc 12
<210> 28
   <211> 4
   <212> PRT
   <213> Artificial Sequence
<220>
   <223> Flexible peptide
<400> 28
   Gly Ser Ser Gly
   1
<210> 29
   <211> 18
   <212> DNA
   <213> Artificial Sequence
<220>
   <223> Flexible peptide
<400> 29
   ggcagcggcg gcggcagc 18
<210> 30
   <211> 6
   <212> PRT
   <213> Artificial Sequence
<220>
   <223> Flexible pept ide
<400> 30
   Gly Ser Gly Gly Gly Ser
   1 5
<210> 31
   <211> 24
   <212> DNA
   <213> Homo sapiens
<400> 31
   ggcaccctgg tgaccgtgag cagc 24
<210> 32
   <211> 8
   <212> PRT
   <213> Homo sapiens
<400> 32
   Gly Thr Leu Val Thr Val Ser Ser
   1 5
<210> 33
   <211> 21
   <212> DNA
   <213> Artificial Sequence
<220>
   <223> T-->A substitution to limit O-linked glycosylation
<400> 33
   accctggtgg cggtgagcag c 21
<210> 34
   <211> 7
   <212> PRT
   <213> Artificial Sequence
<220>
   <223> T-->A substitution to limit O-linked glycosylation
<400> 34
   Thr Leu Val Ala Val Ser Ser
   1 5
<210> 35
   <211> 45
   <212> DNA
   <213> Homo sapiens
<400> 35
   gaaccgaaaa gctgcgataa aacccatacc tgcccgccgt gcccg 45
<210> 36
   <211> 15
   <212> PRT
   <213> Homo sapiens
<400> 36
   Glu Pro Lys Ser Cys Asp Lys Thr His Thr Cys Pro Pro Cys Pro
   1 5 10 15
<210> 37
   <211> 45
   <212> DNA
   <213> Homo sapiens
<400> 37
   gaaccgaaaa gcgcggataa aacccatacc tgcccgccgt gcccg 45
<210> 38
   <211> 15
   <212> PRT
   <213> Homo sapiens
<400> 38
   Glu Pro Lys Ser Ala Asp Lys Thr His Thr Cys Pro Pro Cys Pro
   1               5                   10                  15
<210> 39
   <211> 36
   <212> DNA
   <213> Homo sapiens
<400> 39 36
   gaaagcaaat atggcccgcc gtgcccgagc tgcccg 36
<210> 40
   <211> 12
   <212> PRT
   <213>Homo sapiens
<400> 40
   Glu Ser Lys Tyr Gly Pro Pro Cys Pro Ser Cys Pro
   1               5                   10
<210> 41
   <211> 36
   <212> DNA
   <213> Homo sapiens
<400> 41 36
   gaaagcaaat atggcccgcc gtgcccgccg tgcccg 36
<210> 42
   <211> 12
   <212> PRT
   <213> Homo sapiens
<400> 42
   Glu Ser Lys Tyr Gly Pro Pro Cys Pro Pro Cys Pro
   1               5                   10
<210> 43
   <211> 15
   <212> DNA
   <213> Homo sapiens
<400> 43
   tgcccgccgt gcccg 15
<210> 44
   <211> 5
   <212> PRT
   <213> Homo sapiens
<400> 44
   Cys Pro Pro Cys Pro
   1 5
<210> 45
   <211> 12
   <212> DNA
   <213> Homo sapiens
<400> 45
   tgcccgagct gc 12
<210> 46
   <211> 4
   <212> PRT
   <213> Homo sapiens
<400> 46
   Cys Pro Ser Cys
   1
<210> 47
   <211> 330
   <212> DNA
   <213> Homo sapiens
<400> 47
   gcgccggaac tgctgggcgg cccgagcgtg tttctgtttc cgccgaaacc gaaagatacc 60
   ctgatgatta gccgcacccc ggaagtgacc tgcgtggtgg tggatgtgag ccatgaagat 120
   ccggaagtga aatttaactg gtatgtggat ggcgtggaag tgcataacgc gaaaaccaaa 180
   ccgcgcgaag aacagtataa cagcacctat cgcgtggtga gcgtgctgac cgtgctgcat 240
   caggattggc tgaacggcaa agaatataaa tgcaaagtga gcaacaaagc gctgccggcg 300
   ccgattgaaa aaaccattag caaagcgaaa 330
<210> 48
   <211> 110
   <212> PRT
   <213> Homo sapiens
<400> 48
   Ala Pro Glu Leu Leu Gly Gly Pro Ser Val Phe Leu Phe Pro Pro Lys
   1 5 10 15
   Pro Lys Asp Thr Leu Met Ile Ser Arg Thr Pro Glu Val Thr Cys Val
   20 25 30
   Val Val Asp Val Ser His Glu Asp Pro Glu Val Lys Phe Asn Trp Tyr
   35 40 45
   Val Asp Gly Val Glu Val His Asn Ala Lys Thr Lys Pro Arg Glu Glu
   50 55 60
   Gln Tyr Asn Ser Thr Tyr Arg Val Val Ser Val Leu Thr Val Leu His
   65 70 75 80
   Gln Asp Trp Leu Asn Gly Lys Glu Tyr Lys Cys Lys Val Ser Asn Lys
   85 90 95
   Ala Leu Pro Ala Pro Ile Glu Lys Thr Ile Ser Lys Ala Lys
   100 105 110
<210> 49
   <211> 330
   <212> DNA
   <213> Homo sapiens
<400> 49
   gcgccggaag cggcgggcgg cccgagcgtg tttctgtttc cgccgaaacc gaaagatacc 60
   ctgatgatta gccgcacccc ggaagtgacc tgcgtggtgg tggatgtgag ccatgaagat 120
   ccggaagtga aatttaactg gtatgtggat ggcgtggaag tgcataacgc gaaaaccaaa 180
   ccgcgcgaag aacagtataa cagcacctat cgcgtggtga gcgtgctgac cgtgctgcat 240
   caggattggc tgaacggcaa agaatataaa tgcaaagtga gcaacaaagc gctgccggcg 300
   ccgattgaaa aaaccattag caaagcgaaa 330
<210> 50
   <211> 110
   <212> PRT
   <213> Homo sapiens
<400> 50
   Ala Pro Glu Ala Ala Gly Gly Pro Ser Val Phe Leu Phe Pro Pro Lys
   1 5 10 15
   Pro Lys Asp Thr Leu Met Ile Ser Arg Thr Pro Glu Val Thr Cys Val
   20 25 30
   Val Val Asp Val Ser His Glu Asp Pro Glu Val Lys Phe Asn Trp Tyr
   35 40 45
   Val Asp Gly Val Glu Val His Asn Ala Lys Thr Lys Pro Arg Glu Glu
   50 55 60
   Gln Tyr Asn Ser Thr Tyr Arg Val Val Ser Val Leu Thr Val Leu His
   65 70 75 80
   Gln Asp Trp Leu Asn Gly Lys Glu Tyr Lys Cys Lys Val Ser Asn Lys
   85 90 95
   Ala Leu Pro Ala Pro Ile Glu Lys Thr Ile Ser Lys Ala Lys
   100 105 110
<210> 51
   <211> 330
   <212> DNA
   <213> Homo sapiens
<400> 51
   gcgccggaat ttctgggcgg cccgagcgtg tttctgtttc cgccgaaacc gaaagatacc 60
   ctgatgatta gccgcacccc ggaagtgacc tgcgtggtgg tggatgtgag ccaggaagat 120
   ccggaagtgc agtttaactg gtatgtggat ggcgtggaag tgcataacgc gaaaaccaaa 180
   ccgcgcgaag aacagtttaa cagcacctat cgcgtggtga gcgtgctgac cgtgctgcat 240
   caggattggc tgaacggcaa agaatataaa tgcaaagtga gcaacaaagg cctgccgagc 300
   agcattgaaa aaaccattag caaagcgaaa 330
<210> 52
   <211> 110
   <212> PRT
   <213> Homo sapiens
<400> 52
   Ala Pro Glu Phe Leu Gly Gly Pro Ser Val Phe Leu Phe Pro Pro Lys
   1 5 10 15
   Pro Lys Asp Thr Leu Met Ile Ser Arg Thr Pro Glu Val Thr Cys Val
   20 25 30
   Val Val Asp Val Ser Gln Glu Asp Pro Glu Val Gln Phe Asn Trp Tyr
   35 40 45
   Val Asp Gly Val Glu Val His Asn Ala Lys Thr Lys Pro Arg Glu Glu
   50 55 60
   Gln Phe Asn Ser Thr Tyr Arg Val Val Ser Val Leu Thr Val Leu His
   65 70 75 80
   Gln Asp Trp Leu Asn Gly Lys Glu Tyr Lys Cys Lys Val Ser Asn Lys
   85 90 95
   Gly Leu Pro Ser Ser Ile Glu Lys Thr Ile Ser Lys Ala Lys
   100 105 110
<210> 53
   <211> 330
   <212> DNA
   <213> Homo sapiens
<400> 53
   gcgccggaag cggcgggcgg cccgagcgtg tttctgtttc cgccgaaacc gaaagatacc 60
   ctgatgatta gccgcacccc ggaagtgacc tgcgtggtgg tggatgtgag ccaggaagat 120
   ccggaagtgc agtttaactg gtatgtggat ggcgtggaag tgcataacgc gaaaaccaaa 180
   ccgcgcgaag aacagtttaa cagcacctat cgcgtggtga gcgtgctgac cgtgctgcat 240
   caggattggc tgaacggcaa agaatataaa tgcaaagtga gcaacaaagg cctgccgagc 300
   agcattgaaa aaaccattag caaagcgaaa 330
<210> 54
   <211> 110
   <212> PRT
   <213> Homo sapiens
<400> 54
   Ala Pro Glu Ala Ala Gly Gly Pro Ser Val Phe Leu Phe Pro Pro Lys
   1 5 10 15
   Pro Lys Asp Thr Leu Met Ile Ser Arg Thr Pro Glu Val Thr Cys Val
   20 25 30
   Val Val Asp Val Ser Gln Glu Asp Pro Glu Val Gln Phe Asn Trp Tyr
   35 40 45
   Val Asp Gly Val Glu Val His Asn Ala Lys Thr Lys Pro Arg Glu Glu
   50 55 60
   Gln Phe Asn Ser Thr Tyr Arg Val Val Ser Val Leu Thr Val Leu His
   65 70 75 80
   Gln Asp Trp Leu Asn Gly Lys Glu Tyr Lys Cys Lys Val Ser Asn Lys
   85 90 95
   Gly Leu Pro Ser Ser Ile Glu Lys Thr Ile Ser Lys Ala Lys
   100 105 110
<210> 55
   <211> 321
   <212> DNA
   <213> Homo sapiens
<400> 55
   ggccagccgc gcgaaccgca ggtgtatacc ctgccgccga gccgcgatga actgaccaaa 60
   aaccaggtga gcctgacctg cctggtgaaa ggcttttatc cgagcgatat tgcggtggaa 120
   tgggaaagca acggccagcc ggaaaacaac tataaaacca ccccgccggt gctggatagc 180
   gatggcagct ttttcctgta tagcaaactg accgtggata aaagccgctg gcagcagggc 240
   aacgtgttta gctgcagcgt gatgcatgaa gcgctgcata accattatac ccagaaaagc 300
   ctgagcctga gcccgggcaa a 321
<210> 56
   <211> 107
   <212> PRT
   <213> Homo sapiens
<400> 56
   Gly Gln Pro Arg Glu Pro Gln Val Tyr Thr Leu Pro Pro Ser Arg Asp
   1               5                   10                  15
   Glu Leu Thr Lys Asn Gln Val Ser Leu Thr Cys Leu Val Lys Gly Phe
               20                  25                  30
   Tyr Pro Ser Asp Ile Ala Val Glu Trp Glu Ser Asn Gly Gln Pro Glu
           35                  40                  45
   Asn Asn Tyr Lys Thr Thr Pro Pro Val Leu Asp Ser Asp Gly Ser Phe
       50                  55                  60
   Phe Leu Tyr Ser Lys Leu Thr Val Asp Lys Ser Arg Trp Gln Gln Gly
   65                  70                  75                  80
   Asn Val Phe Ser Cys Ser Val Met His Glu Ala Leu His Asn His Tyr
                   85                  90                  95
   Thr Gln Lys Ser Leu Ser Leu Ser Pro Gly Lys
               100                 105
<210> 57
   <211> 321
   <212> DNA
   <213> Homo sapiens
<400> 57
   ggccagccgc gcgaaccgca ggtgtatacc ctgccgccga gccaggaaga aatgaccaaa 60
   aaccaggtga gcctgacctg cctggtgaaa ggcttttatc cgagcgatat tgcggtggaa 120
   tgggaaagca acggccagcc ggaaaacaac tataaaacca ccccgccggt gctggatagc 180
   gatggcagct tttttctgta tagccgcctg accgtggata aaagccgctg gcaggaaggc 240
   aacgtgttta gctgcagcgt gatgcatgaa gcgctgcata accattatac ccagaaaagc 300
   ctgagcctga gcctgggcaa a 321
<210> 58
   <211> 107
   <212> PRT
   <213> Homo sapiens
<400> 58
   Gly Gln Pro Arg Glu Pro Gln Val Tyr Thr Leu Pro Pro Ser Gln Glu
   1               5                   10                  15
   Glu Met Thr Lys Asn Gln Val Ser Leu Thr Cys Leu Val Lys Gly Phe
   20 25 30
   Tyr Pro Ser Asp Ile Ala Val Glu Trp Glu Ser Asn Gly Gln Pro Glu
   35 40 45
   Asn Asn Tyr Lys Thr Thr Pro Pro Val Leu Asp Ser Asp Gly Ser Phe
   50 55 60
   Phe Leu Tyr Ser Arg Leu Thr Val Asp Lys Ser Arg Trp Gln Glu Gly
   65 70 75 80
   Asn Val Phe Ser Cys Ser Val Met His Glu Ala Leu His Asn His Tyr
   85 90 95
   Thr Gln Lys Ser Leu Ser Leu Ser Leu Gly Lys
   100 105
<210> 59
   <211> 777
   <212> DNA
   <213> Artificial Sequence
<220>
   <223> Melanocortin receptor binding alpha-MSH mimetibody without
   <223> secretory signal.
<400> 59
   tcctactcca tggagcactt ccgctggggc aagccggtgg gatccggtgg aggctccggt 60
   accttagtca ccgtctcctc agagcccaaa tcttgtgaca aaactcacac gtgcccaccg 120
   tgcccagcac ctgaactcct ggggggaccg tcagtcttcc tcttcccccc aaaacccaag 180
   gacaccctca tgatctcccg gacccctgag gtcacatgcg tggtggtgga cgtgagccac 240
   gaagaccctg aggtcaagtt caactggtac gtggacggcg tggaggtgca taatgccaag 300
   acaaagccgc gggaggagca gtacaacagc acgtaccggg tggtcagcgt cctcaccgtc 360
   ctgcaccagg actggctgaa tggcaaggag tacaagtgca aggtctccaa caaagccctc 420
   ccagccccca tcgagaaaac catctccaaa gccaaagggc agccccgaga accacaggtg 480
   tacaccctgc ccccatcccg ggatgagctg accaagaacc aggtcagcct gacctgcctg 540
   gtcaaaggct tctatcccag cgacatcgcc gtggagtggg agagcaatgg gcagccggag 600
   aacaactaca agaccacgcc tcccgtgctg gactccgacg gctccttctt cctctacagc 660
   aagctcaccg tggacaagag caggtggcag caggggaacg tcttctcatg ctccgtgatg 720
   catgaggctc tgcacaacca ctacacgcag aagagcctct ccctgtctcc gggtaaa 777
<210> 60
   <211> 259
   <212> PRT
   <213> Artificial Sequence
<220>
   <223> Melanocortin receptor binding alpha-MSH mimetibody without
   <223> secretory signal.
<400> 60
   Ser Tyr Ser Met Glu His Phe Arg Trp Gly Lys Pro Val Gly Ser Gly
   1               5                   10                  15
   Gly Gly Ser Gly Thr Leu Val Thr Val Ser Ser Glu Pro Lys Ser Cys
               20                  25                  30
   Asp Lys Thr His Thr Cys Pro Pro Cys Pro Ala Pro Glu Leu Leu Gly
           35                  40                  45
   Gly Pro Ser Val Phe Leu Phe Pro Pro Lys Pro Lys Asp Thr Leu Met
       50                  55                  60
   Ile Ser Arg Thr Pro Glu Val Thr Cys Val Val Val Asp Val Ser His
   65                  70                  75                  80
   Glu Asp Pro Glu Val Lys Phe Asn Trp Tyr Val Asp Gly Val Glu Val
                   85                  90                  95
   His Asn Ala Lys Thr Lys Pro Arg Glu Glu Gln Tyr Asn Ser Thr Tyr
               100                 105                 110
   Arg Val Val Ser Val Leu Thr Val Leu His Gln Asp Trp Leu Asn Gly
           115                 120                 125
   Lys Glu Tyr Lys Cys Lys Val Ser Asn Lys Ala Leu Pro Ala Pro Ile
       130                 135                 140
   Glu Lys Thr Ile Ser Lys Ala Lys Gly Gln Pro Arg Glu Pro Gln Val
   145                 150                 155                 160
   Tyr Thr Leu Pro Pro Ser Arg Asp Glu Leu Thr Lys Asn Gln Val Ser
                   165                 170                 175
   Leu Thr Cys Leu Val Lys Gly Phe Tyr Pro Ser Asp Ile Ala Val Glu
               180                 185                 190
   Trp Glu Ser Asn Gly Gln Pro Glu Asn Asn Tyr Lys Thr Thr Pro Pro
           195                 200                 205
   Val Leu Asp Ser Asp Gly Ser Phe Phe Leu Tyr Ser Lys Leu Thr Val
       210                 215                 220
   Asp Lys Ser Arg Trp Gln Gln Gly Asn Val Phe Ser Cys Ser Val Met
   225                 230                 235                 240
   His Glu Ala Leu His Asn His Tyr Thr Gln Lys Ser Leu Ser Leu Ser
                   245                 250                 255
   Pro Gly Lys
<210> 61
   <211> 843
   <212> DNA
   <213> Artificial Sequence
<220>
   <223> Melanocortin receptor binding alpha-MSH mimetibody with secretory
   <223> signal and V1.
<400> 61
   atggcttggg tgtggacctt gctattcctg atggcggccg cccaaagtat acaggcccag     60
   atccagtcct actccatgga gcacttccgc tggggcaagc cggtgggatc cggtggaggc    120
   tccggtacct tagtcaccgt ctcctcagag cccaaatctt gtgacaaaac tcacacgtgc    180
   ccaccgtgcc cagcacctga actcctgggg ggaccgtcag tcttcctctt ccccccaaaa    240
   cccaaggaca ccctcatgat ctcccggacc cctgaggtca catgcgtggt ggtggacgtg    300
   agccacgaag accctgaggt caagttcaac tggtacgtgg acggcgtgga ggtgcataat    360
   gccaagacaa agccgcggga ggagcagtac aacagcacgt accgggtggt cagcgtcctc    420
   accgtcctgc accaggactg gctgaatggc aaggagtaca agtgcaaggt ctccaacaaa    480
   gccctcccag cccccatcga gaaaaccatc tccaaagcca aagggcagcc ccgagaacca    540
   caggtgtaca ccctgccccc atcccgggat gagctgacca agaaccaggt cagcctgacc    600
   tgcctggtca aaggcttcta tcccagcgac atcgccgtgg agtgggagag caatgggcag    660
   ccggagaaca actacaagac cacgcctccc gtgctggact ccgacggctc cttcttcctc    720
   tacagcaagc tcaccgtgga caagagcagg tggcagcagg ggaacgtctt ctcatgctcc    780
   gtgatgcatg aggctctgca caaccactac acgcagaaga gcctctccct gtctccgggt    840
   aaa                                                                  843
<210> 62
   <211> 281
   <212> PRT
   <213> Artificial Sequence
<220>
   <223> Melanocortin receptor binding alpha-MSH mimetibody with secretory <223> signal and V1.
<400> 62
   Met Ala Trp Val Trp Thr Leu Leu Phe Leu Met Ala Ala Ala Gln Ser
   1               5                   10                  15
   Ile Gln Ala Gln Ile Gln Ser Tyr Ser Met Glu His Phe Arg Trp Gly
               20                  25                  30
   Lys Pro Val Gly Ser Gly Gly Gly Ser Gly Thr Leu Val Thr Val Ser
           35                  40                  45
   Ser Glu Pro Lys Ser Cys Asp Lys Thr His Thr Cys Pro Pro Cys Pro
       50                  55                  60
   Ala Pro Glu Leu Leu Gly Gly Pro Ser Val Phe Leu Phe Pro Pro Lys
   65                  70                  75                  80
   Pro Lys Asp Thr Leu Met Ile Ser Arg Thr Pro Glu Val Thr Cys Val
                   85                  90                  95
   Val Val Asp Val Ser His Glu Asp Pro Glu Val Lys Phe Asn Trp Tyr
               100                 105                 110
   Val Asp Gly Val Glu Val His Asn Ala Lys Thr Lys Pro Arg Glu Glu
           115                 120                 125
   Gln Tyr Asn Ser Thr Tyr Arg Val Val Ser Val Leu Thr Val Leu His
       130                 135                 140
   Gln Asp Trp Leu Asn Gly Lys Glu Tyr Lys Cys Lys Val Ser Asn Lys
   145                 150                 155                 160
   Ala Leu Pro Ala Pro Ile Glu Lys Thr Ile Ser Lys Ala Lys Gly Gln
                   165                 170                 175
   Pro Arg Glu Pro Gln Val Tyr Thr Leu Pro Pro Ser Arg Asp Glu Leu
               180                 185                 190
   Thr Lys Asn Gln Val Ser Leu Thr Cys Leu Val Lys Gly Phe Tyr Pro
           195                 200                 205
   Ser Asp Ile Ala Val Glu Trp Glu Ser Asn Gly Gln Pro Glu Asn Asn
       210                 215                 220
   Tyr Lys Thr Thr Pro Pro Val Leu Asp Ser Asp Gly Ser Phe Phe Leu
   225                 230                 235                 240
   Tyr Ser Lys Leu Thr Val Asp Lys Ser Arg Trp Gln Gln Gly Asn Val
   245 250 255
   Phe Ser Cys Ser Val Met His Glu Ala Leu His Asn His Tyr Thr Gln
   260 265 270
   Lys Ser Leu Ser Leu Ser Pro Gly Lys
   275 280
<210> 63
   <211> 11978
   <212> DNA
   <213> Artificial Sequence
<220>
   <223> Expression vector.
<400> 63
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc tttttattaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcccag atccagtcct actccatgga 1680
   gcacttccgc tggggcaagc cggtgggatc cggtggaggc tccggtacct tagtcaccgt 1740
   ctcctcagag cccaaatctt gtgacaaaac tcacacgtgc ccaccgtgcc cagcacctga 1800
   actcctgggg ggaccgtcag tcttcctctt ccccccaaaa cccaaggaca ccctcatgat 1860
   ctcccggacc cctgaggtca catgcgtggt ggtggacgtg agccacgaag accctgaggt 1920
   caagttcaac tggtacgtgg acggcgtgga ggtgcataat gccaagacaa agccgcggga 1980
   ggagcagtac aacagcacgt accgggtggt cagcgtcctc accgtcctgc accaggactg 2040
   gctgaatggc aaggagtaca agtgcaaggt ctccaacaaa gccctcccag cccccatcga 2100
   gaaaaccatc tccaaagcca aagggcagcc ccgagaacca caggtgtaca ccctgccccc 2160
   atcccgggat gagctgacca agaaccaggt cagcctgacc tgcctggtca aaggcttcta 2220
   tcccagcgac atcgccgtgg agtgggagag caatgggcag ccggagaaca actacaagac 2280
   cacgcctccc gtgctggact ccgacggctc cttcttcctc tacagcaagc tcaccgtgga 2340
   caagagcagg tggcagcagg ggaacgtctt ctcatgctcc gtgatgcatg aggctctgca 2400
   caaccactac acgcagaaga gcctctccct gtctccgggt aaatgagggc ccgtttaaac 2460
   agatccagac atgataagat acattgatga gtttggacaa accacaacta gaatgcagtg 2520
   aaaaaaatgc tttatttgtg aaatttgtga tgctattgct ttatttgtaa ccattataag 2580
   ctgcaataaa caagttaaca acaacaattg cattcatttt atgtttcagg ttcaggggga 2640
   ggtgtgggag gttttttaaa gcaagtaaaa cctctacaaa tgtggtatgg ctgattatga 2700
   tccggctgcc tcgcgcgttt cggtgatgac ggtgaaaacc tctgacacat gcagctcccg 2760
   gagacggtca cagcttgtct gtaagcggat gccgggagca gacaagcccg tcaggcgtca 2820
   gcgggtgttg gcgggtgtcg gggcgcagcc atgaggtcga ctctagagga tcgatgcccc 2880
   gccccggacg aactaaacct gactacgaca tctctgcccc ttcttcgcgg ggcagtgcat 2940
   gtaatccctt cagttggttg gtacaacttg ccaactgggc cctgttccac atgtgacacg 3000
   gggggggacc aaacacaaag gggttctctg actgtagttg acatccttat aaatggatgt 3060
   gcacatttgc caacactgag tggctttcat cctggagcag actttgcagt ctgtggactg 3120
   caacacaaca ttgcctttat gtgtaactct tggctgaagc tcttacacca atgctggggg 3180
   acatgtacct cccaggggcc caggaagact acgggaggct acaccaacgt caatcagagg 3240
   ggcctgtgta gctaccgata agcggaccct caagagggca ttagcaatag tgtttataag 3300
   gcccccttgt taaccctaaa cgggtagcat atgcttcccg ggtagtagta tatactatcc 3360
   agactaaccc taattcaata gcatatgtta cccaacggga agcatatgct atcgaattag 3420
   ggttagtaaa agggtcctaa ggaacagcga tatctcccac cccatgagct gtcacggttt 3480
   tatttacatg gggtcaggat tccacgaggg tagtgaacca ttttagtcac aagggcagtg 3540
   gctgaagatc aaggagcggg cagtgaactc tcctgaatct tcgcctgctt cttcattctc 3600
   cttcgtttag ctaatagaat aactgctgag ttgtgaacag taaggtgtat gtgaggtgct 3660
   cgaaaacaag gtttcaggtg acgcccccag aataaaattt ggacgggggg ttcagtggtg 3720
   gcattgtgct atgacaccaa tataaccctc acaaacccct tgggcaataa atactagtgt 3780
   aggaatgaaa cattctgaat atctttaaca atagaaatcc atggggtggg gacaagccgt 3840
   aaagactgga tgtccatctc acacgaattt atggctatgg gcaacacata atcctagtgc 3900
   aatatgatac tggggttatt aagatgtgtc ccaggcaggg accaagacag gtgaaccatg 3960
   ttgttacact ctatttgtaa caaggggaaa gagagtggac gccgacagca gcggactcca 4020
   ctggttgtct ctaacacccc cgaaaattaa acggggctcc acgccaatgg ggcccataaa 4080
   caaagacaag tggccactct tttttttgaa attgtggagt gggggcacgc gtcagccccc 4140
   acacgccgcc ctgcggtttt ggactgtaaa ataagggtgt aataacttgg ctgattgtaa 4200
   ccccgctaac cactgcggtc aaaccacttg cccacaaaac cactaatggc accccgggga 4260
   atacctgcat aagtaggtgg gcgggccaag ataggggcgc gattgctgcg atctggagga 4320
   caaattacac acacttgcgc ctgagcgcca agcacagggt tgttggtcct catattcacg 4380
   aggtcgctga gagcacggtg ggctaatgtt gccatgggta gcatatacta cccaaatatc 4440
   tggatagcat atgctatcct aatctatatc tgggtagcat aggctatcct aatctatatc 4500
   tgggtagcat atgctatcct aatctatatc tgggtagtat atgctatcct aatttatatc 4560
   tgggtagcat aggctatcct aatctatatc tgggtagcat atgctatcct aatctatatc 4620
   tgggtagtat atgctatcct aatctgtatc cgggtagcat atgctatcct aatagagatt 4680
   agggtagtat atgctatcct aatttatatc tgggtagcat atactaccca aatatctgga 4740
   tagcatatgc tatcctaatc tatatctggg tagcatatgc tatcctaatc tatatctggg 4800
   tagcataggc tatcctaatc tatatctggg tagcatatgc tatcctaatc tatatctggg 4860
   tagtatatgc tatcctaatt tatatctggg tagcataggc tatcctaatc tatatctggg 4920
   tagcatatgc tatcctaatc tatatctggg tagtatatgc tatcctaatc tgtatccggg 4980
   tagcatatgc tatcctcatg catatacagt cagcatatga tacccagtag tagagtggga 5040
   gtgctatcct ttgcatatgc cgccacctcc caagggggcg tgaattttcg ctgcttgtcc 5100
   ttttcctgct ggttgctccc attcttaggt gaatttaagg aggccaggct aaagccgtcg 5160
   catgtctgat tgctcaccag gtaaatgtcg ctaatgtttt ccaacgcgag aaggtgttga 5220
   gcgcggagct gagtgacgtg acaacatggg tatgcccaat tgccccatgt tgggaggacg 5280
   aaaatggtga caagacagat ggccagaaat acaccaacag cacgcatgat gtctactggg 5340
   gatttattct ttagtgcggg ggaatacacg gcttttaata cgattgaggg cgtctcctaa 5400
   caagttacat cactcctgcc cttcctcacc ctcatctcca tcacctcctt catctccgtc 5460
   atctccgtca tcaccctccg cggcagcccc ttccaccata ggtggaaacc agggaggcaa 5520
   atctactcca tcgtcaaagc tgcacacagt caccctgata ttgcaggtag gagcgggctt 5580
   tgtcataaca aggtccttaa tcgcatcctt caaaacctca gcaaatatat gagtttgtaa 5640
   aaagaccatg aaataacaga caatggactc ccttagcggg ccaggttgtg ggccgggtcc 5700
   aggggccatt ccaaagggga gacgactcaa tggtgtaaga cgacattgtg gaatagcaag 5760
   ggcagttcct cgccttaggt tgtaaaggga ggtcttacta cctccatata cgaacacacc 5820
   ggcgacccaa gttccttcgt cggtagtcct ttctacgtga ctcctagcca ggagagctct 5880
   taaaccttct gcaatgttct caaatttcgg gttggaacct ccttgaccac gatgcttttc 5940
   caaaccaccc tccttttttg cgccctgcct ccatcaccct gaccccgggg tccagtgctt 6000
   gggccttctc ctgggtcatc tgcggggccc tgctctatcg ctcccggggg cacgtcaggc 6060
   tcaccatctg ggccaccttc ttggtggtat tcaaaataat cggcttcccc tacagggtgg 6120
   aaaaatggcc ttctacctgg agggggcctg cgcggtggag acccggatga tgatgactga 6180
   ctactgggac tcctgggcct cttttctcca cgtccacgac ctctccccct ggctctttca 6240
   cgacttcccc ccctggctct ttcacgtcct ctaccccggc ggcctccact acctcctcga 6300
   ccccggcctc cactacctcc tcgaccccgg cctccactgc ctcctcgacc ccggcctcca 6360
   cctcctgctc ctgcccctcc tgctcctgcc cctcctcctg ctcctgcccc tcctgcccct 6420
   cctgctcctg cccctcctgc ccctcctgct cctgcccctc ctgcccctcc tgctcctgcc 6480
   cctcctgccc ctcctcctgc tcctgcccct cctgcccctc ctcctgctcc tgcccctcct 6540
   gcccctcctg ctcctgcccc tcctgcccct cctgctcctg cccctcctgc ccctcctgct 6600
   cctgcccctc ctgctcctgc ccctcctgct cctgcccctc ctgctcctgc ccctcctgcc 6660
   cctcctgccc ctcctcctgc tcctgcccct cctgctcctg cccctcctgc ccctcctgcc 6720
   cctcctgctc ctgcccctcc tcctgctcct gcccctcctg cccctcctgc ccctcctcct 6780
   gctcctgccc ctcctgcccc tcctcctgct cctgcccctc ctcctgctcc tgcccctcct 6840
   gcccctcctg cccctcctcc tgctcctgcc cctcctgccc ctcctcctgc tcctgcccct 6900
   cctcctgctc ctgcccctcc tgcccctcct gcccctcctc ctgctcctgc ccctcctcct 6960
   gctcctgccc ctcctgcccc tcctgcccct cctgcccctc ctcctgctcc tgcccctcct 7020
   cctgctcctg cccctcctgc tcctgcccct cccgctcctg ctcctgctcc tgttccaccg 7080
   tgggtccctt tgcagccaat gcaacttgga cgtttttggg gtctccggac accatctcta 7140
   tgtcttggcc ctgatcctga gccgcccggg gctcctggtc ttccgcctcc tcgtcctcgt 7200
   cctcttcccc gtcctcgtcc atggttatca ccccctcttc tttgaggtcc actgccgccg 7260
   gagccttctg gtccagatgt gtctcccttc tctcctaggc catttccagg tcctgtacct 7320
   ggcccctcgt cagacatgat tcacactaaa agagatcaat agacatcttt attagacgac 7380
   gctcagtgaa tacagggagt gcagactcct gccccctcca acagcccccc caccctcatc 7440
   cccttcatgg tcgctgtcag acagatccag gtctgaaaat tccccatcct ccgaaccatc 7500
   ctcgtcctca tcaccaatta ctcgcagccc ggaaaactcc cgctgaacat cctcaagatt 7560
   tgcgtcctga gcctcaagcc aggcctcaaa ttcctcgtcc ccctttttgc tggacggtag 7620
   ggatggggat tctcgggacc cctcctcttc ctcttcaagg tcaccagaca gagatgctac 7680
   tggggcaacg gaagaaaagc tgggtgcggc ctgtgaggat cagcttatcg atgataagct 7740
   gtcaaacatg agaattcttg aagacgaaag ggcctcgtga tacgcctatt tttataggtt 7800
   aatgtcatga taataatggt ttcttagacg tcaggtggca cttttcgggg aaatgtgcgc 7860
   ggaaccccta tttgtttatt tttctaaata cattcaaata tgtatccgct catgagacaa 7920
   taaccctgat aaatgcttca ataatattga aaaaggaaga gtatgagtat tcaacatttc 7980
   cgtgtcgccc ttattccctt ttttgcggca ttttgccttc ctgtttttgc tcacccagaa 8040
   acgctggtga aagtaaaaga tgctgaagat cagttgggtg cacgagtggg ttacatcgaa 8100
   ctggatctca acagcggtaa gatccttgag agttttcgcc ccgaagaacg ttttccaatg 8160
   atgagcactt ttaaagttct gctatgtggc gcggtattat cccgtgttga cgccgggcaa 8220
   gagcaactcg gtcgccgcat acactattct cagaatgact tggttgagta ctcaccagtc 8280
   acagaaaagc atcttacgga tggcatgaca gtaagagaat tatgcagtgc tgccataacc 8340
   atgagtgata acactgcggc caacttactt ctgacaacga tcggaggacc gaaggagcta 8400
   accgcttttt tgcacaacat gggggatcat gtaactcgcc ttgatcgttg ggaaccggag 8460
   ctgaatgaag ccataccaaa cgacgagcgt gacaccacga tgcctgcagc aatggcaaca 8520
   acgttgcgca aactattaac tggcgaacta cttactctag cttcccggca acaattaata 8580
   gactggatgg aggcggataa agttgcagga ccacttctgc gctcggccct tccggctggc 8640
   tggtttattg ctgataaatc tggagccggt gagcgtgggt ctcgcggtat cattgcagca 8700
   ctggggccag atggtaagcc ctcccgtatc gtagttatct acacgacggg gagtcaggca 8760
   actatggatg aacgaaatag acagatcgct gagataggtg cctcactgat taagcattgg 8820
   taactgtcag accaagttta ctcatatata ctttagattg atttaaaact tcatttttaa 8880
   tttaaaagga tctaggtgaa gatccttttt gataatctca tgaccaaaat cccttaacgt 8940
   gagttttcgt tccactgagc gtcagacccc gtagaaaaga tcaaaggatc ttcttgagat 9000
   cctttttttc tgcgcgtaat ctgctgcttg caaacaaaaa aaccaccgct accagcggtg 9060
   gtttgtttgc cggatcaaga gctaccaact ctttttccga aggtaactgg cttcagcaga 9120
   gcgcagatac caaatactgt ccttctagtg tagccgtagt taggccacca cttcaagaac 9180
   tctgtagcac cgcctacata cctcgctctg ctaatcctgt taccagtggc tgctgccagt 9240
   ggcgataagt cgtgtcttac cgggttggac tcaagacgat agttaccgga taaggcgcag 9300
   cggtcgggct gaacgggggg ttcgtgcaca cagcccagct tggagcgaac gacctacacc 9360
   gaactgagat acctacagcg tgagctatga gaaagcgcca cgcttcccga agggagaaag 9420
   gcggacaggt atccggtaag cggcagggtc ggaacaggag agcgcacgag ggagcttcca 9480
   gggggaaacg cctggtatct ttatagtcct gtcgggtttc gccacctctg acttgagcgt 9540
   cgatttttgt gatgctcgtc aggggggcgg agcctatgga aaaacgccag caacgcggcc 9600
   tttttacggt tcctggcctt ttgctggcct tgaagctgtc cctgatggtc gtcatctacc 9660
   tgcctggaca gcatggcctg caacgcgggc atcccgatgc cgccggaagc gagaagaatc 9720
   ataatgggga aggccatcca gcctcgcgtc gcgaacgcca gcaagacgta gcccagcgcg 9780
   tcggccccga gatgcgccgc gtgcggctgc tggagatggc ggacgcgatg gatatgttct 9840
   gccaagggtt ggtttgcgca ttcacagttc tccgcaagaa ttgattggct ccaattcttg 9900
   gagtggtgaa tccgttagcg aggtgccgcc ctgcttcatc cccgtggccc gttgctcgcg 9960
   tttgctggcg gtgtccccgg aagaaatata tttgcatgtc tttagttcta tgatgacaca 10020
   aaccccgccc agcgtcttgt cattggcgaa ttcgaacacg cagatgcagt cggggcggcg 10080
   cggtccgagg tccacttcgc atattaaggt gacgcgtgtg gcctcgaaca ccgagcgacc 10140
   ctgcagcgac ccgcttaaca gcgtcaacag cgtgccgcag atcccggggg gcaatgagat 10200
   atgaaaaagc ctgaactcac cgcgacgtct gtcgagaagt ttctgatcga aaagttcgac 10260
   agcgtctccg acctgatgca gctctcggag ggcgaagaat ctcgtgcttt cagcttcgat 10320
   gtaggagggc gtggatatgt cctgcgggta aatagctgcg ccgatggttt ctacaaagat 10380
   cgttatgttt atcggcactt tgcatcggcc gcgctcccga ttccggaagt gcttgacatt 10440
   ggggaattca gcgagagcct gacctattgc atctcccgcc gtgcacaggg tgtcacgttg 10500
   caagacctgc ctgaaaccga actgcccgct gttctgcagc cggtcgcgga ggccatggat 10560
   gcgatcgctg cggccgatct tagccagacg agcgggttcg gcccattcgg accgcaagga 10620
   atcggtcaat acactacatg gcgtgatttc atatgcgcga ttgctgatcc ccatgtgtat 10680
   cactggcaaa ctgtgatgga cgacaccgtc agtgcgtccg tcgcgcaggc tctcgatgag   10740
   ctgatgcttt gggccgagga ctgccccgaa gtccggcacc tcgtgcacgc ggatttcggc   10800
   tccaacaatg tcctgacgga caatggccgc ataacagcgg tcattgactg gagcgaggcg   10860
   atgttcgggg attcccaata cgaggtcgcc aacatcttct tctggaggcc gtggttggct   10920
   tgtatggagc agcagacgcg ctacttcgag cggaggcatc cggagcttgc aggatcgccg   10980
   cggctccggg cgtatatgct ccgcattggt cttgaccaac tctatcagag cttggttgac   11040
   ggcaatttcg atgatgcagc ttgggcgcag ggtcgatgcg acgcaatcgt ccgatccgga   11100
   gccgggactg tcgggcgtac acaaatcgcc cgcagaagcg cggccgtctg gaccgatggc   11160
   tgtgtagaag tactcgccga tagtggaaac cgacgcccca gcactcgtcc ggatcgggag   11220
   atgggggagg ctaactgaaa cacggaagga gacaataccg gaaggaaccc gcgctatgac   11280
   ggcaataaaa agacagaata aaacgcacgg gtgttgggtc gtttgttcat aaacgcgggg   11340
   ttcggtccca gggctggcac tctgtcgata ccccaccgag accccattgg ggccaatacg   11400
   cccgcgtttc ttccttttcc ccaccccacc ccccaagttc gggtgaaggc ccagggctcg   11460
   cagccaacgt cggggcggca ggccctgcca tagccactgg ccccgtgggt tagggacggg   11520
   gtcccccatg gggaatggtt tatggttcgt gggggttatt attttgggcg ttgcgtgggg   11580
   tcaggtccac gactggactg agcagacaga cccatggttt ttggatggcc tgggcatgga   11640
   ccgcatgtac tggcgcgaca cgaacaccgg gcgtctgtgg ctgccaaaca cccccgaccc   11700
   ccaaaaacca ccgcgcggat ttctggcgtg ccaagctagt cgaccaattc tcatgtttga   11760
   cagcttatca tcgcagatcc gggcaacgtt gttgccattg ctgcaggcgc agaactggta   11820
   ggtatggaag atctatacat tgaatcaata ttggcaatta gccatattag tcattggtta   11880
   tatagcataa atcaatattg gctattggcc attgcatacg ttgtatctat atcataatat   11940
   gtacatttat attggctcat gtccaatatg accgccat                           11978
<210> 64
   <211> 696
   <212> DNA
   <213> Homo sapiens
<400> 64
   gaaccgaaaa gctgcgataa aacccatacc tgcccgccgt gcccggcgcc ggaactgctg 60
   ggcggcccga gcgtgtttct gtttccgccg aaaccgaaag ataccctgat gattagccgc 120
   accccggaag tgacctgcgt ggtggtggat gtgagccatg aagatccgga agtgaaattt 180
   aactggtatg tggatggcgt ggaagtgcat aacgcgaaaa ccaaaccgcg cgaagaacag 240
   tataacagca cctatcgcgt ggtgagcgtg ctgaccgtgc tgcatcagga ttggctgaac 300
   ggcaaagaat ataaatgcaa agtgagcaac aaagcgctgc cggcgccgat tgaaaaaacc 360
   attagcaaag cgaaaggcca gccgcgcgaa ccgcaggtgt ataccctgcc gccgagccgc 420
   gatgaactga ccaaaaacca ggtgagcctg acctgcctgg tgaaaggctt ttatccgagc 480
   gatattgcgg tggaatggga aagcaacggc cagccggaaa acaactataa aaccaccccg 540
   ccggtgctgg atagcgatgg cagctttttt ctgtatagca aactgaccgt ggataaaagc 600
   cgctggcagc agggcaacgt gtttagctgc agcgtgatgc atgaagcgct gcataaccat 660
   tatacccaga aaagcctgag cctgagcccg ggcaaa 696
<210> 65
   <211> 232
   <212> PRT
   <213> Homo sapiens
<400> 65
   Glu Pro Lys Ser Cys Asp Lys Thr His Thr Cys Pro Pro Cys Pro Ala
   1 5 10 15
   Pro Glu Leu Leu Gly Gly Pro Ser Val Phe Leu Phe Pro Pro Lys Pro
   20 25 30
   Lys Asp Thr Leu Met Ile Ser Arg Thr Pro Glu Val Thr Cys Val Val
   35 40 45
   Val Asp Val Ser His Glu Asp Pro Glu Val Lys Phe Asn Trp Tyr Val
   50 55 60
   Asp Gly Val Glu Val His Asn Ala Lys Thr Lys Pro Arg Glu Glu Gln
   65 70 75 80
   Tyr Asn Ser Thr Tyr Arg Val Val Ser Val Leu Thr Val Leu His Gln
   85 90 95
   Asp Trp Leu Asn Gly Lys Glu Tyr Lys Cys Lys Val Ser Asn Lys Ala
   100 105 110
   Leu Pro Ala Pro Ile Glu Lys Thr Ile Ser Lys Ala Lys Gly Gln Pro
   115 120 125
   Arg Glu Pro Gln Val Tyr Thr Leu Pro Pro Ser Arg Asp Glu Leu Thr
   130 135 140
   Lys Asn Gln Val Ser Leu Thr Cys Leu Val Lys Gly Phe Tyr Pro Ser
   145 150 155 160
   Asp Ile Ala Val Glu Trp Glu Ser Asn Gly Gln Pro Glu Asn Asn Tyr
   165 170 175
   Lys Thr Thr Pro Pro Val Leu Asp Ser Asp Gly Ser Phe Phe Leu Tyr
   180 185 190
   Ser Lys Leu Thr Val Asp Lys Ser Arg Trp Gln Gln Gly Asn Val Phe
   195 200 205
   Ser Cys Ser Val Met His Glu Ala Leu His Asn His Tyr Thr Gln Lys
   210 215 220
   Ser Leu Ser Leu Ser Pro Gly Lys
   225 230
<210> 66
   <211> 45
   <212>DNA
   <213 > Homo sapiens
<400> 66
   atgattagcc gcaccccgac cgtgctgcat cagcataacc attat 45
<210> 67
   <211> 15
   <212> PRT
   <213 > Homo sapiens
<400> 67
   Met Ile Ser Arg Thr Pro Thr Val Leu His Gln His Asn His Tyr
   1 5 10 15
<210> 68
   <211> 57
   <212> DNA
   <213> Homo sapiens
<400> 68
   atggcttggg tgtggacctt gctattcctg atggcggccg cccaaagtat acaggcc 57
<210> 69
   <211> 19
   <212> PRT
   <213> Homo sapiens
<400> 69
   Met Ala Trp Val Trp Thr Leu Leu Phe Leu Met Ala Ala Ala Gln Ser
   1 5 10 15
   Ile Gln Ala
<210> 70
   <211> 951
   <212> DNA
   <213> Homo sapiens
<400> 70
   atggctgtgc agggatccca gagaagactt ctgggctccc tcaactccac ccccacagcc      60
   atcccccagc tggggctggc tgccaaccag acaggagccc ggtgcctgga ggtgtccatc     120
   tctgacgggc tcttcctcag cctggggctg gtgagcttgg tggagaacgc gctggtggtg     180
   gccaccatcg ccaagaaccg gaacctgcac tcacccatgt actgcttcat ctgctgcctg     240
   gccttgtcgg acctgctggt gagcgggagc aacgtgctgg agacggccgt catcctcctg     300
   ctggaggccg gtgcactggt ggcccgggct gcggtgctgc agcagctgga caatgtcatt     360
   gacgtgatca cctgcagctc catgctgtcc agcctctgct tcctgggcgc catcgccgtg     420
   gaccgctaca tctccatctt ctacgcactg cgctaccaca gcaccgtgac cctgccgcgg     480
   gcgcggcgag ccgttgcggc catctgggtg gccagtgtcg tcttcagcac gctcttcatc     540
   gcctactacg accacgtggc cgtcctgctg tgcctcgtgg tcttcttcct ggctatgctg     600
   gtgctcatgg ccgtgctgta cgtccacatg ctggcccggg cctgccagca cgcccagggc     660
   atcgcccggc tccacaagag gcagcgcccg gtccaccagg gctttggcct taaaggcgct     720
   gtcaccctca ccatcctgct gggcattttc ttcctctgct ggggcccctt cttcctgcat     780
   ctcacactca tcgtcctctg ccccgagcac cccacgtgcg gctgcatctt caagaacttc     840
   aacctctttc tcgccctcat catctgcaat gccatcatcg accccctcat ctacgccttc     900
   cacagccagg agctccgcag gacgctcaag gaggtgctga cgtgctcctg g              951
<210> 71
   <211> 317
   <212> PRT
   <213> Homo sapiens
<400> 71
   Met Ala Val Gln Gly Ser Gln Arg Arg Leu Leu Gly Ser Leu Asn Ser
   1               5                   10                  15
   Thr Pro Thr Ala Ile Pro Gln Leu Gly Leu Ala Ala Asn Gln Thr Gly
               20                  25                  30
   Ala Arg Cys Leu Glu Val Ser Ile Ser Asp Gly Leu Phe Leu Ser Leu
           35                  40                  45
   Gly Leu Val Ser Leu Val Glu Asn Ala Leu Val Val Ala Thr Ile Ala
       50                  55                  60
   Lys Asn Arg Asn Leu His Ser Pro Met Tyr Cys Phe Ile Cys Cys Leu
   65                  70                  75                  80
   Ala Leu Ser Asp Leu Leu Val Ser Gly Ser Asn Val Leu Glu Thr Ala
                   85                      90                  95
   Val Ile Leu Leu Leu Glu Ala Gly Ala Leu Val Ala Arg Ala Ala Val
               100                 105                 110
   Leu Gln Gln Leu Asp Asn Val Ile Asp Val Ile Thr Cys Ser Ser Met
           115                 120                 125
   Leu Ser Ser Leu Cys Phe Leu Gly Ala Ile Ala Val Asp Arg Tyr Ile
       130                 135                 140
   Ser Ile Phe Tyr Ala Leu Arg Tyr His Ser Thr Val Thr Leu Pro Arg
   145                 150                 155                 160
   Ala Arg Arg Ala Val Ala Ala Ile Trp Val Ala Ser Val Val Phe Ser
                   165                 170                 175
   Thr Leu Phe Ile Ala Tyr Tyr Asp His Val Ala Val Leu Leu Cys Leu
               180                 185                 190
   Val Val Phe Phe Leu Ala Met Leu Val Leu Met Ala Val Leu Tyr Val
           195                 200                 205
   His Met Leu Ala Arg Ala Cys Gln His Ala Gln Gly Ile Ala Arg Leu
       210                 215                 220
   His Lys Arg Gln Arg Pro Val His Gln Gly Phe Gly Leu Lys Gly Ala
   225                 230                 235                 240
   Val Thr Leu Thr Ile Leu Leu Gly Ile Phe Phe Leu Cys Trp Gly Pro
                   245                 250                 255
   Phe Phe Leu His Leu Thr Leu Ile Val Leu Cys Pro Glu His Pro Thr
               260                 265                 270
   Cys Gly Cys Ile Phe Lys Asn Phe Asn Leu Phe Leu Ala Leu Ile Ile
           275                 280                 285
   Cys Asn Ala Ile Ile Asp Pro Leu Ile Tyr Ala Phe His Ser Gln Glu
       290                 295                 300
   Leu Arg Arg Thr Leu Lys Glu Val Leu Thr Cys Ser Trp
   305                 310                 315
<210> 72
   <211> 891
   <212> DNA
   <213> Homo sapiens
<400> 72
   atgaagcaca ttatcaactc gtatgaaaac atcaacaaca cagcaagaaa taattccgac     60
   tgtcctcgtg tggttttgcc ggaggagata tttttcacaa tttccattgt tggagttttg    120
   gagaatctga tcgtcctgct ggctgtgttc aagaataaga atctccaggc acccatgtac    180
   tttttcatct gtagcttggc catatctgat atgctgggca gcctatataa gatcttggaa    240
   aatatcctga tcatattgag aaacatgggc tatctcaagc cacgtggcag ttttgaaacc    300
   acagccgatg acatcatcga ctccctgttt gtcctctccc tgcttggctc catcttcagc    360
   ctgtctgtga ttgctgcgga ccgctacatc accatcttcc acgcactgcg gtaccacagc    420
   atcgtgacca tgcgccgcac tgtggtggtg cttacggtca tctggacgtt ctgcacgggg    480
   actggcatca ccatggtgat cttctcccat catgtgccca cagtgatcac cttcacgtcg    540
   ctgttcccgc tgatgctggt cttcatcctg tgcctctatg tgcacatgtt cctgctggct    600
   cgatcccaca ccaggaagat ctccaccctc cccagagcca acatgaaagg ggccatcaca    660
   ctgaccatcc tgctcggggt cttcatcttc tgctgggccc cctttgtgct tcatgtcctc    720
   ttgatgacat tctgcccaag taacccctac tgcgcctgct acatgtctct cttccaggtg    780
   aacggcatgt tgatcatgtg caatgccgtc attgacccct tcatatatgc cttccggagc    840
   ccagagctca gggacgcatt caaaaagatg atcttctgca gcaggtactg g              891
<210> 73
   <211> 297
   <212> PRT
   <213> Homo sapiens
<400> 73
<210> 74
   <211> 1080
   <212> DNA
   <213> Homo sapiens
<400> 74
   atgagcatcc aaaagacgta tctggaggga gattttgtct ttcctgtgag cagcagcagc 60
   ttcctacgga ccctgctgga gccccagctc ggatcagccc ttctgacagc aatgaatgct 120
   tcgtgctgcc tgccctctgt tcagccaaca ctgcctaatg gctcggagca cctccaagcc 180
   cctttcttca gcaaccagag cagcagcgcc ttctgtgagc aggtcttcat caagcccgag 240
   gttttcctgt ctctgggcat cgtcagtctg ctggaaaaca tcctggttat cctggccgtg 300
   gtcaggaacg gcaacctgca ctccccgatg tacttctttc tctgcagcct ggcggtggcc 360
   gacatgctgg taagtgtgtc caatgccctg gagaccatca tgatcgccat cgtccacagc 420
   gactacctga ccttcgagga ccagtttatc cagcacatgg acaacatctt cgactccatg 480
   atctgcatct ccctggtggc ctccatctgc aacctcctgg ccatcgccgt cgacaggtac 540
   gtcaccatct tttacgcgct ccgctaccac agcatcatga ccgtgaggaa ggccctcacc 600
   ttgatcgtgg ccatctgggt ctgctgcggc gtctgtggcg tggtgttcat cgtctactcg 660
   gagagcaaaa tggtcattgt gtgcctcatc accatgttct tcgccatgat gctcctcatg 720
   ggcaccctct acgtgcacat gttcctcttt gcgcggctgc acgtcaagcg catagcagca 780
   ctgccacctg ccgacggggt ggccccacag caacactcat gcatgaaggg ggcagtcacc 840
   atcaccattc tcctgggcgt gttcatcttc tgctgggccc ccttcttcct ccacctggtc 900
   ctcatcatca cctgccccac caacccctac tgcatctgct acactgccca cttcaacacc 960
   tacctggtcc tcatcatgtg caactccgtc atcgacccac tcatctacgc tttccggagc 1020
   ctggaattgc gcaacacctt tagggagatt ctctgtggct gcaacggcat gaacttggga 1080
<210> 75
   <211> 360
   <212> PRT
   <213> Homo sapiens
<400> 75
   Met Ser Ile Gln Lys Thr Tyr Leu Glu Gly Asp Phe Val Phe Pro Val
   1               5                   10                  15
   Ser Ser Ser Ser Phe Leu Arg Thr Leu Leu Glu Pro Gln Leu Gly Ser
               20                  25                  30
   Ala Leu Leu Thr Ala Met Asn Ala Ser Cys Cys Leu Pro Ser Val Gln
           35                  40                  45
   Pro Thr Leu Pro Asn Gly Ser Glu His Leu Gln Ala Pro Phe Phe Ser
       50                  55                  60
   Asn Gln Ser Ser Ser Ala Phe Cys Glu Gln Val Phe Ile Lys Pro Glu
   65                  70                  75                  80
   Val Phe Leu Ser Leu Gly Ile Val Ser Leu Leu Glu Asn Ile Leu Val
   85 90 95
   Ile Leu Ala Val Val Arg Asn Gly Asn Leu His Ser Pro Met Tyr Phe
   100 105 110
   Phe Leu Cys Ser Leu Ala Val Ala Asp Met Leu Val Ser Val Ser Asn
   115 120 125
   Ala Leu Glu Thr Ile Met Ile Ala Ile Val His Ser Asp Tyr Leu Thr
   130 135 140
   Phe Glu Asp Gln Phe Ile Gln His Met Asp Asn Ile Phe Asp Ser Met
   145 150 155 160
   Ile Cys Ile Ser Leu Val Ala Ser Ile Cys Asn Leu Leu Ala Ile Ala
   165 170 175
   Val Asp Arg Tyr Val Thr Ile Phe Tyr Ala Leu Arg Tyr His Ser Ile
   180 185 190
   Met Thr Val Arg Lys Ala Leu Thr Leu Ile Val Ala Ile Trp Val Cys
   195 200 205
   Cys Gly Val Cys Gly Val Val Phe Ile Val Tyr Ser Glu Ser Lys Met
   210 215 220
   Val Ile Val Cys Leu Ile Thr Met Phe Phe Ala Met Met Leu Leu Met
   225 230 235 240
   Gly Thr Leu Tyr Val His Met Phe Leu Phe Ala Arg Leu His Val Lys
   245 250 255
   Arg Ile Ala Ala Leu Pro Pro Ala Asp Gly Val Ala Pro Gln Gln His
   260 265 270
   Ser Cys Met Lys Gly Ala Val Thr Ile Thr Ile Leu Leu Gly Val Phe
   275 280 285
   Ile Phe Cys Trp Ala Pro Phe Phe Leu His Leu Val Leu Ile Ile Thr
   290 295 300
   Cys Pro Thr Asn Pro Tyr Cys Ile Cys Tyr Thr Ala His Phe Asn Thr
   305 310 315 320
   Tyr Leu Val Leu Ile Met Cys Asn Ser Val Ile Asp Pro Leu Ile Tyr
   325 330 335
   Ala Phe Arg Ser Leu Glu Leu Arg Asn Thr Phe Arg Glu Ile Leu Cys
   340 345 350
   Gly Cys Asn Gly Met Asn Leu Gly
   355 360
<210> 76
   <211> 999
   <212> DNA
   <213> Homo sapiens
<400> 76
   atggtgaact ccacccaccg tgggatgcac acttctctgc acctctggaa ccgcagcagt 60
   tacagactgc acagcaatgc cagtgagtcc cttggaaaag gctactctga tggagggtgc 120
   tacgagcaac tttttgtctc tcctgaggtg tttgtgactc tgggtgtcat cagcttgttg 180
   gagaatatct tagtgattgt ggcaatagcc aagaacaaga atctgcattc acccatgtac 240
   tttttcatct gcagcttggc tgtggctgat atgctggtga gcgtttcaaa tggatcagaa 300
   accattatca tcaccctatt aaacagtaca gatacggatg cacagagttt cacagtgaat 360
   attgataatg tcattgactc ggtgatctgt agctccttgc ttgcatccat ttgcagcctg 420
   ctttcaattg cagtggacag gtactttact atcttctatg ctctccagta ccataacatt 480
   atgacagtta agcgggttgg gatcatcata agttgtatct gggcagcttg cacggtttca 540
   ggcattttgt tcatcattta ctcagatagt agtgctgtca tcatctgcct catcaccatg 600
   ttcttcacca tgctggctct catggcttct ctctatgtcc acatgttcct gatggccagg 660
   cttcacatta agaggattgc tgtcctcccc ggcactggtg ccatccgcca aggtgccaat 720
   atgaagggag cgattacctt gaccatcctg attggcgtct ttgttgtctg ctgggcccca 780
   ttcttcctcc acttaatatt ctacatctct tgtcctcaga atccatattg tgtgtgcttc 840
   atgtctcact ttaacttgta tctcatactg atcatgtgta attcaatcat cgatcctctg 900
   atttatgcac tccggagtca agaactgagg aaaaccttca aagagatcat ctgttgctat 960
   cccctgggag gcctttgtga cttgtctagc agatattaa 999
<210> 77
   <211> 332
   <212> PRT
   <213> Homo sapiens
<400> 77
   Met Val Asn Ser Thr His Arg Gly Met His Thr Ser Leu His Leu Trp
   1 5 10 15
   Asn Arg Ser Ser Tyr Arg Leu His Ser Asn Ala Ser Glu Ser Leu Gly
               20                  25                  30
   Lys Gly Tyr Ser Asp Gly Gly Cys Tyr Glu Gln Leu Phe Val Ser Pro
           35                  40                  45
   Glu Val Phe Val Thr Leu Gly Val Ile Ser Leu Leu Glu Asn Ile Leu
       50                  55                  60
   Val Ile Val Ala Ile Ala Lys Asn Lys Asn Leu His Ser Pro Met Tyr
   65                  70                  75                  80
   Phe Phe Ile Cys Ser Leu Ala Val Ala Asp Met Leu Val Ser Val Ser
                   85                  90                  95
   Asn Gly Ser Glu Thr Ile Ile Ile Thr Leu Leu Asn Ser Thr Asp Thr
               100                 105                 110
   Asp Ala Gln Ser Phe Thr Val Asn Ile Asp Asn Val Ile Asp Ser Val
           115                 120                 125
   Ile Cys Ser Ser Leu Leu Ala Ser Ile Cys Ser Leu Leu Ser Ile Ala
       130                 135                 140
   Val Asp Arg Tyr Phe Thr Ile Phe Tyr Ala Leu Gln Tyr His Asn Ile
   145                 150                 155                 160
   Met Thr Val Lys Arg Val Gly Ile Ile Ile Ser Cys Ile Trp Ala Ala
                   165                 170                 175
   Cys Thr Val Ser Gly Ile Leu Phe Ile Ile Tyr Ser Asp Ser Ser Ala
               180                 185                 190
   Val Ile Ile Cys Leu Ile Thr Met Phe Phe Thr Met Leu Ala Leu Met
           195                 200                 205
   Ala Ser Leu Tyr Val His Met Phe Leu Met Ala Arg Leu His Ile Lys
       210                 215                 220
   Arg Ile Ala Val Leu Pro Gly Thr Gly Ala Ile Arg Gln Gly Ala Asn
   225                 230                 235                 240
   Met Lys Gly Ala Ile Thr Leu Thr Ile Leu Ile Gly Val Phe Val Val
                   245                 250                 255
   Cys Trp Ala Pro Phe Phe Leu His Leu Ile Phe Tyr Ile Ser Cys Pro
               260                 265                 270
   Gln Asn Pro Tyr Cys Val Cys Phe Met Ser His Phe Asn Leu Tyr Leu
   275 280 285
   Ile Leu Ile Met Cys Asn Ser Ile Ile Asp Pro Leu Ile Tyr Ala Leu
   290 295 300
   Arg Ser Gln Glu Leu Arg Lys Thr Phe Lys Glu Ile Ile Cys Cys Tyr
   305 310 315 320
   Pro Leu Gly Gly Leu Cys Asp Leu Ser Ser Arg Tyr
   325 330
<210> 78
   <211> 975
   <212> DNA
   <213> Homo sapiens
<400> 78
   atgaattcct catttcacct gcatttcttg gatctcaacc tgaatgccac agagggcaac 60
   ctttcaggac ccaatgtcaa aaacaagtct tcaccatgtg aagacatggg cattgctgtg 120
   gaggtgtttc tcactctggg tgtcatcagc ctcttggaga acatcttggt cataggggcc 180
   atagtgaaga acaaaaacct gcactccccc atgtacttct tcgtgtgcag cctggcagtg 240
   gcggacatgc tggtgagcat gtccagtgcc tgggagacca tcaccatcta cctactcaac 300
   aacaagcacc tagtgatagc agacgccttt gtgcgccaca ttgacaatgt gtttgactcc 360
   atgatctgca tttccgtggt ggcatccatg tgcagcttac tggccattgc agtggatagg 420
   tacgtcacca tcttctacgc cctgcgctac caccacatca tgacggcgag gcgctcaggg 480
   gccatcatcg ccggcatctg ggctttctgc acgggctgcg gcattgtctt catcctgtac 540
   tcagaatcca cctacgtcat cctgtgcctc atctccatgt tcttcgctat gctgttcctc 600
   ctggtgtctc tgtacataca catgttcctc ctggcgcgga ctcacgtcaa gcggatcgcg 660
   gctctgcccg gggccagctc tgcgcggcag aggaccagca tgcagggcgc ggtcaccgtc 720
   accatgctgc tgggcgtgtt taccgtgtgc tgggccccgt tcttccttca tctcacttta 780
   atgctttctt gccctcagaa cctctactgc tctcgcttca tgtctcactt caatatgtac 840
   ctcatactca tcatgtgtaa ttccgtgatg gaccctctca tatatgcctt ccgcagccaa 900
   gagatgcgga agacctttaa ggagattatt tgctgccgtg gtttcaggat cgcctgcagc 960
   tttcccagaa gggat 975
<210> 79
   <211> 325
   <212> PRT
   <213> Homo sapiens
<400> 79
   Met Asn Ser Ser Phe His Leu His Phe Leu Asp Leu Asn Leu Asn Ala
   1               5                   10                  15
   Thr Glu Gly Asn Leu Ser Gly Pro Asn Val Lys Asn Lys Ser Ser Pro
               20                  25                  30
   Cys Glu Asp Met Gly Ile Ala Val Glu Val Phe Leu Thr Leu Gly Val
           35                  40                  45
   Ile Ser Leu Leu Glu Asn Ile Leu Val Ile Gly Ala Ile Val Lys Asn
       50                  55                  60
   Lys Asn Leu His Ser Pro Met Tyr Phe Phe Val Cys Ser Leu Ala Val
   65                  70                  75                  80
   Ala Asp Met Leu Val Ser Met Ser Ser Ala Trp Glu Thr Ile Thr Ile
                   85                  90                  95
   Tyr Leu Leu Asn Asn Lys His Leu Val Ile Ala Asp Ala Phe Val Arg
               100                 105                 110
   His Ile Asp Asn Val Phe Asp Ser Met Ile Cys Ile Ser Val Val Ala
           115                 120                 125
   Ser Met Cys Ser Leu Leu Ala Ile Ala Val Asp Arg Tyr Val Thr Ile
       130                 135                 140
   Phe Tyr Ala Leu Arg Tyr His His Ile Met Thr Ala Arg Arg Ser Gly
   145                 150                 155                 160
   Ala Ile Ile Ala Gly Ile Trp Ala Phe Cys Thr Gly Cys Gly Ile Val
                   165                 170                 175
   Phe Ile Leu Tyr Ser Glu Ser Thr Tyr Val Ile Leu Cys Leu Ile Ser
               180                 185                 190
   Met Phe Phe Ala Met Leu Phe Leu Leu Val Ser Leu Tyr Ile His Met
           195                 200                 205
   Phe Leu Leu Ala Arg Thr His Val Lys Arg Ile Ala Ala Leu Pro Gly
       210                 215                 220
   Ala Ser Ser Ala Arg Gln Arg Thr Ser Met Gln Gly Ala Val Thr Val
   225                 230                 235                 240
   Thr Met Leu Leu Gly Val Phe Thr Val Cys Trp Ala Pro Phe Phe Leu
   245 250 255
   His Leu Thr Leu Met Leu Ser Cys Pro Gln Asn Leu Tyr Cys Ser Arg
   260 265 270
   Phe Met Ser His Phe Asn Met Tyr Leu Ile Leu Ile Met Cys Asn Ser
   275 280 285
   Val Met Asp Pro Leu Ile Tyr Ala Phe Arg Ser Gln Glu Met Arg Lys
   290 295 300
   Thr Phe Lys Glu Ile Ile Cys Cys Arg Gly Phe Arg Ile Ala Cys Ser
   305 310 315 320
   Phe Pro Arg Arg Asp
   325
<210> 80
   <211> 12
   <212> DNA
   <213> Homo sapiens
<400> 80
   cattttcgct gg 12
<210> 81
   <211> 4
   <212> PRT
   <213> Homo sapiens
<400> 81
   His Phe Arg Trp
   1
<210> 82
   <211> 12
   <212> DNA
   <213> Artificial Sequence
<220>
   <223> Modification of alpha-MSH HFRW core.
<400> 82
   tttcattgga tg 12
<210> 83
   <211> 4
   <212> PRT
   <213> Artificial Sequence
<220>
   <223> Modification of alpha-MSH HFRW core.
<400> 83
   Phe His Trp Met
   1
<210> 84
   <211> 12
   <212> DNA
   <213> Artificial Sequence
<220>
   <223> Flexible peptide
<400> 84
   ggcggcggca gc 12
<210> 85
   <211> 4
   <212> PRT
   <213> Artificial Sequence
<220>
   <223> Flexible peptide
<400> 85
   Gly Gly Gly Ser
   1

## Description

### Field of the Invention

The present invention relates to melanocortin receptor binding mimetibodies, polynucleotides encoding these, cells comprising the polynucleotides or expressing the mimetibodies, and methods of making and using the foregoing.

### Background of the Invention

Obesity is a chronic disease manifested by an excess of fat mass in proportion to body size. Today, every third American is considered over-weight (Body Mass Index (BMI) >25 kg/m²), thus prompting the United States Centers for Disease Control and Prevention (CDC) to declare that obesity is reaching epidemic proportions (Cummings and Schwartz, Annu. Rev. Med. 54:453-471((2003)). The importance of treating obesity is emphasized by the fact that this disease is either the underlying cause, or a risk factor, for developing diseases such as Type 2 Diabetes, congestive heart failure, osteoarthritis and sleep apnea among others.

Additionally, obesity is linked to "Metabolic Syndrome" which is a medical condition characterized by obesity, atherogenic dyslipidemia, elevated blood pressure and insulin resistance. Metabolic Syndrome affects an increasing number of people in the United States. Importantly, it has been shown that even a modest decrease in body weight (5-10% of initial body weight) may significantly improve Metabolic Syndrome conditions and decrease the risk factors for developing obesity-associated disease (Wing et al., Arch. Intern. Med. 147:1749-1753 (1987); Tuomilehto et al., New Engl. J. Med. 344:1343-1350 (2001); Knowler et al. , New Engl. J Med. 346:393-403 (2002); Franz et al., Diabetes Care 25:148-198 (2002)). Additionally, treatment of obesity may be important from a mental health perspective due to the social stigma often attached to obese individuals in some cultures.

Melanocortin receptors play a major role in the regulation of overall energy balance and obesity in both humans and rodents Alpha-melanocyte stimulating hormone (alpha-MSH) is a 13 amino acid peptide hormone that is an important component of the melanocortin system. Alpha-MSH is produced by the proteolytic processing of proopiomelanocortin (POMC) released by the pituitary gland. Alpha-MSH binds with high affinity to the melanocortin 4 receptor (MC4R), but also binds melanocortin receptor 3 (MC3R) and melanocortin receptor 5 (MC5R) with lower affinity. MC4R is a G-coupled protein receptor found in the brain which, when stimulated by alpha-MSH binding, causes decreased food intake and increased fat oxidation. Ultimately, stimulation of melanocortin receptors such as MC4R results in weight loss.

In humans and rodents, loss of function mutations in the different components of the melanocortin system are closely correlated with obesity and related conditions. In mice, mutations within POMC, or MC4R and MC3R produce obesity, insulin resistance and hyperphagia (Goodfellow and Saunders, Curr. Topics Med. Chem. 3: 855-883 (2003); Huszar et al., Cell 88:131-141 (1997); Yaswen et al., Nat. Med. 5: 1066-1070 (1999)). In man mutations within POMC or MC4R lead to the development of obesity associated with increased food intake (Krude et al., Nat. Genet. 19:155-157 (1998); Yeo et al., Nature Genetics 20:111-112 (1998); Branson et al., New Engl. J. Med. 348: 1096-1103 (2003); Vaisse et al., J. Clin. Invest. 106):253-262 (2000); Ho and MacKenzie, J. Biol. Chem. 275: 35816-35822 (1999)).

Weight loss can result from the pharmacological stimulation of melanocortin system activity. In rodents pharmacological stimulation of melanocortin receptors such as MC4R leads to decreased food intake, increased energy expenditure and weight loss (Pierroz et al., Diabetes 51: 1337-1345 (2002)). In man the intranasal administration of alpha-MSH to stimulate MC4R in non-obese men results in decreased body weight due to the loss of fat-but not lean body mass (Fehm et al., J. Clin. Endo. Metabol. 86: 1144-1148 (2001)).

Obesity is currently treated, with only limited success, by several different strategies. These strategies primarily involve "life-style" changes (e.g. diet and exercise), small molecule based pharmaceutical therapies or surgical removal of a portion of the stomach (gastric by-pass surgery). Additionally, weight loss stimulating melanocortin receptor binding peptides such as alpha -MSH are of limited use as pharmaceuticals due to the extremely short serum half-life of such peptides. Thus, a need exists for additional obesity treatments and in particular for melanocortin receptor binding molecules that overcome the short serum half-life of melanocortin receptor binding peptides such as alpha-MSH.

### Brief Description of the Drawings

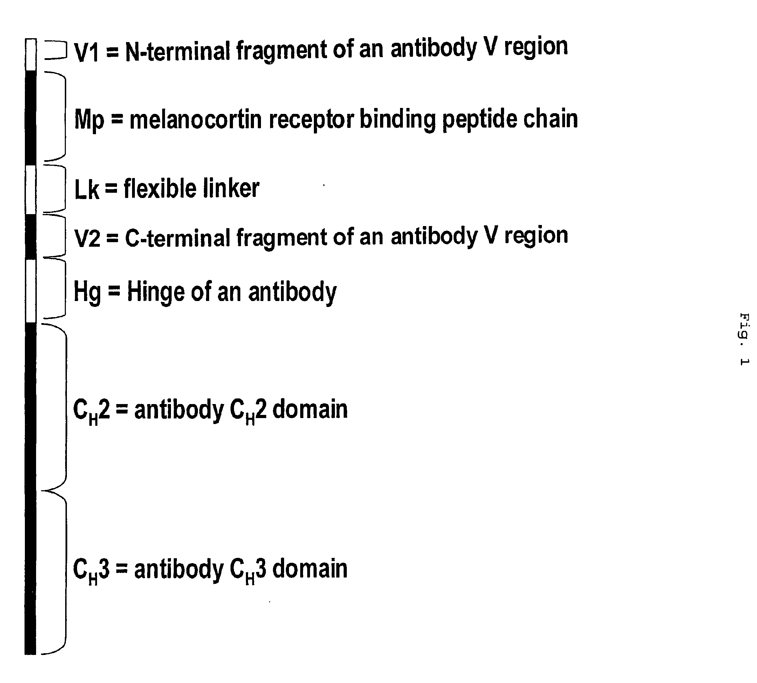

Fig. 1 shows elements of a melanocortin receptor binding mimetibody polypeptide.

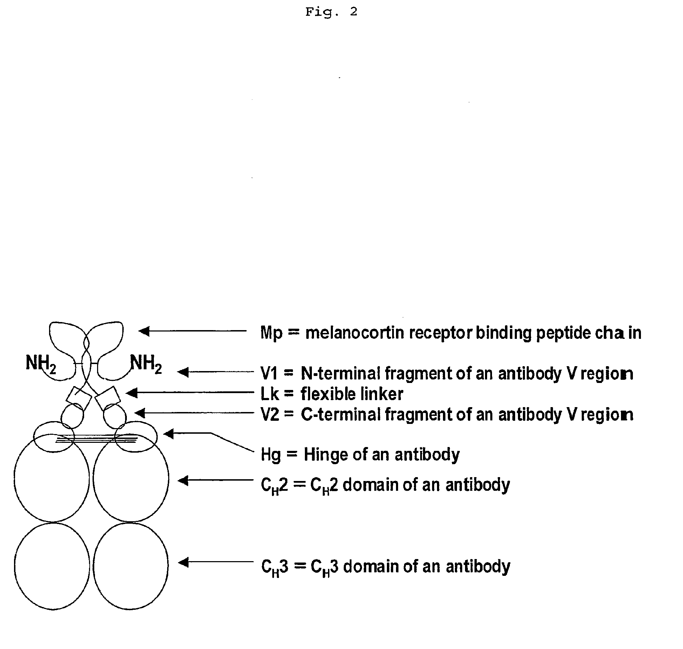

Fig 2 shows a cartoon of a melanocortin receptor binding mimetibody.
Fig. 3 shows the amino acid (SEQ ID NO: 62) and cDNA (SEQ ID NO: 61) sequences of a melanocortin receptor binding alpha-MSH mimetibody. The amino terminal portions of individual mimetibody elements are underlined.
Fig. 4 shows alpha-MSH mimetibody binding to MC4R in a competitive binding assay.
Fig. 5 shows alpha-MSH mimetibody activation of MC4R in cells expressing a high level of MC4R.
Fig. 6 shows alpha-MSH mimetibody activation of MC4R in cells expressing a low level of MC4R.
Fig. 7 shows alpha-MSH mimetibody-mediated decrease in animal food intake.
Fig. 8 shows alpha-MSH mimetibody-mediated decrease in animal body weight.

### Summary of the Invention

One aspect of the invention is a polypeptide according to formula (I):

(Mp-Lk-V2-Hg-C_{H}2-C_{H}3) ₍ₜ₎ (I)

where Mp is a melanocortin receptor binding molecule, Lk is a polypeptide or chemical linkage, V2 is a portion of a C-terminus of an immunoglobulin variable region, Hg is at least a portion of an immunoglobulin variable hinge region, C_{H}2 is an immunoglobulin heavy chain C_{H}2 constant region and C_{H}3 is an immunoglobulin heavy chain C_{H}3 constant region and t is independently an integer from 1 to 10.

Another aspect of the invention is a polypeptide wherein Mp is a biologically active fragment of SEQ ID NO: 2, 4, 6, 8, 10, 12, 14, 16, or 18; or Mp has the amino acid sequence shown in SEQ ID NO: 2, 4, 6, 8, 10, 12, 14, 16, or 18.

Another aspect of the invention is a polypeptide wherein the polypeptide binds to at least one melanocortin receptor.

Another aspect of the invention is a polypeptide comprising SEQ ID NO: 60 or 62.

Another aspect of the invention is a polynucleotide comprising SEQ ID NO: 59 or SEQ ID NO: 61 or a polynucleotide complementary to SEQ ID NO: 59 or SEQ ID NO: 61.

Another aspect of the invention is a polynucleotide comprising a polynucleotide encoding the polypeptide of SEQ ID NO: 60 or SEQ ID NO: 62.

Another aspect of the invention is a polypeptide of the invention for use in medicine.

Another aspect of the invention is a polypeptide of the invention for modulating at least one melanocortin receptor mediated condition.

### Detailed Description of the Invention

The present invention provides polypeptides having the properties of binding a melanocortin receptor and mimicking different isotypes of antibody immunoglobulin molecules such as IgA, IgD, IgE, IgG, or IgM, and any subclass thereof, such as IgA₁, IgA₂, 1₉G₁, IgG₂, IgG₃ or IgG₄, or combinations thereof, herein after generally referred to as "mimetibodies." In some embodiments, the mimetibody polypeptides of the invention contain an alpha melanocyte stimulating hormone peptide (alpha-MSH) sequence and are designated melanocortin receptor blinding alpha-MSH mimetibody. Such alpha-MSH mimetibody polypeptides can bind melanocortin receptor 4 (MC4R) and, with equal and lower affinity, for MC3R and MC5R respectively. One result of such melanocortin receptor binding can be the stimulation or inhibition of melanocortin receptor activity. Stimulation can cause weight loss while inhibition may cause weight gain.

In one embodiment the polypeptides of the invention have the generic formula (I):

(Mp-Lk-V2-Hg-C_{H}2-C_{H3}) ₍ₜ₎ (I)

where Mp is a melanocortin receptor binding molecule, Lk is a polypeptide or chemical linkage, V2 is a portion of a C-terminus of an immunoglobulin variable region, Hg is at least a portion of an immunoglobulin variable hinge region, C_{H}2 is an immunoglobulin heavy chain C_{H}2 constant region and C_{H}3 is an immunoglobulin heavy chain C_{H}3 constant region and t is independently an integer of 1 to 10.

As used herein, "melanocortin receptor binding molecule" means a molecule, which can bind at least one melanocortin receptor such as *Homo sapiens* MC4R (SEQ ID NO: 77). Examples of other *Homo sapiens* melanocortin receptors include MCR1 (SEQ ID NO: 71), MCR2 (SEQ ID NO: 73), MCR3 (SEQ ID NO: 75), and MCR5 (SEQ ID NO: 79). A given peptide chain is a "melanocortin receptor" if it has at least 85% amino acid sequence identity to a known melanocortin receptor sequence or the mature form of a known melanocortin receptor and can function as a G-protein coupled receptor. Percent identity between two peptide chains can be determined by pairwise alignment using the default settings of the AlignX module of Vector NTI v.9.0 .0 (Invitrogen Corp., Carslbad, CA). An exemplary melanocortin receptor binding molecule is the 13 amino acid alpha-MSH peptide having the amino acid sequence shown in (SEQ ID NO: 2). Other melanocortin receptor binding molecules include biologically active fragments of SEQ ID NO: 2 and other amino acid sequences that can bind a melanocortin receptor. The term "biologically active fragment" as used herein, refers to a portion of an alpha-MSH peptide that can bind to a melanocortin receptor such as MC4R. The peptide sequence HFRW (SEQ. ID. NO. 81) is an exemplary "biologically active fragment" of the alpha-MSH peptide sequence SYSMEHFRWGKPV (SEQ ID NO: 2). The HFRW fragment has been incorporated into the structure of the synthetic melanocortin receptor activator molecule melanotan II (MTII) (Fan et al., Nature 385: 165-168 (1997)).

Incorporation of melanocortin receptor binding molecules in the mimetibody polypeptides of the invention provides for binding to melanocortin receptors with a wide range of affinities. The mimetibody polypeptides of the invention may bind a melanocortin receptor with a K_{d} less than or equal to about 10⁻⁷, 10⁻⁸, 10⁻⁹, 10⁻¹⁰, 10⁻¹¹ or 10⁻¹² M. The range of obtained IC50 values for aMSH peptide, MTII peptide and aMSHMMB were 260-400 nM, 5-30 nM and 200-300 nM respectively. The affinity of a mimetibody polypeptide for a melanocortin receptor can be determined experimentally using any suitable method. Such methods may utilize Biacore or KinExA instrumentation, ELISA or competitive binding assays. Mimetibody polypeptides binding specific melanocortin receptors with a desired affinity can be selected from libraries of variants or fragments by techniques known to those skilled in the art.

An alpha-MSH peptide having the amino acid sequence shown in SEQ ID NO: 2 may be modified to obtain other melanocortin receptor binding molecules. Such modifications may comprise the incorporation of C-[X]ₙ-C motifs into the peptide to conformationally constrain the peptide through the formation of disulfide bonds. In a C-[X]ₙ-C motif, C is a cysteine residue, X is a amino acid residues and n is an integer necessary to acheive the required conformational constraint. In this instance n can be as little as 1 residue and as high as 50. Exemplary C-[X]ₙ-C modified peptide sequences are shown in SEQ ID NOs: 4, 6 , 8 and 10.

The modification may also comprise the incorporation of a Wa-[X]ₙ-Wa motif into the peptide to conformationally constrain the peptide through the formation of a tryptophan zipper. In a Wa-[X]ₙ-Wa motif W is tryptophan residue, X is an amino acid, a is an integer usually 2, but can be from 1 to 10, and n is an integer necessary to acheive the required conformational constraint. In this instance n can be as little a 1 residue and as high as 50. Exemplary Wa-[X]ₙ-Wa peptides are shown in SEQ ID NOs: 12, 14, 16 and 18. Further, the sequence HFRW (SEQ ID NO: 81) present in the alpha-MSH peptide may also be modified by substituting any residue in this sequence with any one of F, H, W and M; for example, HFRW (SEQ ID NO: 81) can be substituted to FHWM (SEQ ID NO: 83).

In the polypeptides of the invention, the linker portion (Lk) provides structural flexibility by allowing the mimetibody to have alternative orientations and binding properties. Exemplary linkers include non-peptide chemical linkages or one to 20 amino acids linked by peptide bonds, wherein the amino acids are selected from the 20 naturally occurring amino acids or other amino acids (e.g. D-amino acids, non-naturally occurring amino acids, or rare naturally occuring amino acids). The linker portion can include a majority of amino acids that are sterically unhindered, such as glycine, alanine and serine and can include GS, poly GS (e.g. GSGS (SEQ ID NO: 20)), GGSG (SEQ ID NO: 22), GSGGGS (SEQ ID NO: 24), GSGGGSG (SEQ ID NO: 26), GSSG (SEQ ID NO: 28), or GSGGGS (SEQ ID NO: 30) or GGGS (SEQ ID NO: 85) or any combination or polymer thereof. Other exemplary linkers within the scope of the invention may be longer than 20 residues and may include residues other than glycine, alanine and serine.

In the polypeptides of the invention, V2 is a portion of a carboxy terminal domain of an immunoglobulin variable region such as a heavy chain variable region. Exemplary V2 amino acid sequences are GTLVTVSS (SEQ ID NO: 32) and TLVAVSS (SEQ ID NO: 34).

In the polypeptides of the invention, Hg is a portion of the hinge domain of an immunoglobulin variable region such as a heavy chain variable region. Exemplary Hg amino acid sequences include EPKSCDKTHTCPPCP (SEQ ID NO: 36), EPKSADKTHTCPPCP (SEQ ID NO: 38), ESKYGPPCPSCP (SEQ ID NO: 40), ESKYGPPCPPCP (SEQ ID NO: 42), CPPCP (SEQ ID NO: 44) and CPSC (SEQ ID NO: 46) .

In the polypeptides of the invention, C_{H}2 is an immunoglobulin heavy chain C_{H}2 constant region. Exemplary C_{H}2 amino acid sequences include: and

In the polypeptides of the invention, C_{H}3 is an immunoglobulin heavy chain C_{H}3 constant region. Exemplary C_{H}3 amino acid sequences include: and recognized by those skilled in the art that the C_{H}3 region of the polypeptides of the invention may have its C-terminal amino acid cleaved off when expressed in certain recombinant systems.

In the mimetibody polypeptides of invention Hg, C_{H}2 or C_{H}3 may be of the IgG₁ or IgG₄ subclass. A sequence is of the IgG₁ or IgG₄ subclass if it is formed or developed from a γ1 or y4 heavy chain respectively. A given peptide chain is a γ1 or y4 heavy chain if it is at least 80% identical to a known γ1 or y4 heavy chain sequence of a given species. Percent identity between two peptide chains can be determined by pairwise alignment using the default settings of the AlignX module of Vector NTI v.9.0.0 (Invitrogen Corp., Carlsbad, CA) .

In the mimetibody polypeptides of the invention Hg, C_{H}2 or C_{H}3 may individually be of the IgG₁ or IgG₄ subclass. The mimetibodies of the invention may also comprise combinations of Hg, C_{H}2 or C_{H}3 elements from each subclass For example, Hg may be of the IgG₄ subclass while C_{H}2 and C_{H}3 are of the IgG₁ subclass. Alternatively, Hg, C_{H}2 and C_{H}3 may all of the IgG₄ or IgG₁ subclass. The polypeptide EPKSCDKTHTCPPCPAPELLGGPSVFLFPPKPKDTLMISRT PEVTCVVVDVSHEDPEVKFNWYVDGVE VHNAKTKPREEQYNSTYRVVSVLTVLHQDWLNGKEYKCKVSNKALPAPIEKTISKAKGQPREPQVYTL PPSRDELTKNQVSLTCLVKGFYPSDIAVEWESNGQPENNYKTTPPVLDSDGSFFLYSKLTVDKSRWQQ GNVFSCSVMHEALHNHYTQKSLSLSPGK (SEQ ID NO: 65) is exemplary of a polypeptide in which Hg (residues 1-15 of SEQ ID NO: 65), C_{H}2 (residues 16-125 of SEQ ID NO: 65), and C_{H}3 (residues 126-232 of SEQ ID NO: 65) are all of the IgG₁ subclass.

The IgG₁ and IgG₄ subclasses differ in the number of cysteines in the hinge region. Most IgG type antibodies, such as IgG₁, are homodimeric molecules made up of two identical heavy (H) chains and two identical light (L) chains, typically abbreviated H₂L₂. Thus, these molecules are generally bivalent with respect to antigen binding due to the formation of inter-heavy chain disulfide bonds and both antigen binding (Fab) arms of the IgG molecule have identical binding specificity. IgG₄ isotype heavy chains, in contrast, contain a CPSC (SEQ ID NO: 46) motif in their hinge regions capable of forming either inter- or intra-heavy chain disulfide bonds, *i.e*., the two Cys residues in the CPSC motif may disulfide bond with the corresponding Cys residues in the other H chain (inter) or the two Cys residues within a given CPSC motif may disulfide bond with each other (intra). Since the HL pairs in those IgG₄ molecules with intra-heavy chain bonds in the hinge region are not covalently associated with each other, they may dissociate into HL monomers that then reassociate with HL monomers derived from other IgG₄ molecules forming bispecific, heterodimeric IgG₄ molecules. *In vivo* isomerase enzymes may facilitate this process. In a bispecific IgG antibody the two Fab "arms" of the antibody molecule differ in the epitopes that they bind. Substituting Ser residues in the hinge region of IgG₄ with Pro results in "IgG₁-like behavior," *i.e*., the molecules form stable disulfide bonds between heavy chains and therefore, are not susceptible to HL exchange with other IgG₄ molecules.

The mimetibody polypeptides of the invention may be made more IgG₄-like, or IgG₁-like by the modification of sites which are involved in disulfide bond formation and are present in the Hg-C_{H}2-C_{H}3 portion of the mimetibody polypeptides. Such sites may be modified by removal, deletion, insertion or substitution with other amino acids. Typically, the cysteine residues present in disulfide bond associated motifs are removed or substituted. Removal of these sites may avoid covalent disulfide bonding with other cysteine-containing proteins present in the mimetibody producing host cell or intra-heavy chain disulfide bonding in IgG₄-based constructs while still allowing for noncovalent dimerization of mimetibody Hg-C_{H}2-C_{H}3 domains. Modification of such sites can permit the formation of bispecific mimetibody polypeptides with two different M portions or prevent the formation of such bispecific species.

The IgG₁ and IgG₄ subclasses also differ in their ability to mediate complement dependent cytotoxicity (CDC) and antibody-dependent cellular cytotoxicity (ADCC). CDC is the lysing of a target cell in the presence of complement. The complement activation pathway is initiated by the binding of the first component of the complement system (C1q) to a molecule complexed with a cognate antigen. IgG₁ is a strong inducer of the complement cas cade and subsequent CDC activity, while IgG₄ has little complement-inducing activity. ADCC is a cell-mediated process in which nonspecific cytotoxic cells that express Fc receptors (FcRs) involved in ADCC (*e.g*., natural killer (NK) cells, neutrophils, and macrophages) recognize bound antibody on a target cell and subsequently cause lysis of the target cell. The IgG₁ subclass binds with high affinity to Fc receptors involved in ADCC and contributes to ADCC, while IgG₄ binds only weakly to such receptors and has little ADCC inducing activity. The relative inability of IgG₄ to activate effector functions such as ADCC is desirable since delivery of the mimetibody polypeptide to cells without cell killing is possible.

The CDC and ADCC activity of the mimetibody polypeptides of the invention may be modified by altering sites involved in CDC and ADCC present in the Hg-C_{H}2-C_{H}3 portion of the mimetibody polypeptide. Such sites may be modified by removal, deletion, insertion or substitution with other amino acids. In the mimetibodies of the invention sites involved in CDC, such as the C1q binding site, are typically removed or otherwise modified to minimize CDC activity. Additionally, Fc receptor binding sites involved in ADCC can also be similarly modified in the mimetibodies of the invention. In general, such modification will remove Fc receptor binding sites involved in ADCC activity from the mimetibodies of the invention. The substitution of Leu residues with Ala residues in the C_{H}2 portion of the polypeptides of the invention is one example of a modification which can minimize ADCC activity in the polypeptides of the invention. The C_{H}2 amino acid sequence APEAAGGPSVFLFPPKPKDTLMISRTPEVTCVVVDVSHED PEVKFNWYVDGVEVHNAKTKPREEQYNS TYRVVSVLTVLHQDWLNGKEYKCKVSNKALPAPIEKTISKAK (SEQ ID NO: 52) is exemplary of such a Leu to Ala substitution at residues 4 and 5 (in sequence above). Further, the V1 domain can be removed such that the N-terminus of the peptide is free following cleavage of the signal peptide, and is accessible to and could be modified by enzymes such as acetylases.

Antibodies of both the IgG₄ and IgG₁ isotypes contain FcRn salvage receptor binding sites. The FcRn salvage receptor helps maintain IgG antibody levels in the body by recycling or transporting IgG type antibodies across enodothelial cell layers such as those lining the inside of body cavities and blood vessels.

The FcRn salavage receptor does this by binding IgGs that have entered endothelial cells by nonspecific pinocytosis and preventing these IgG antibody molecules from being degraded in the lysosome of the cell. The result of such FcRn receptor activity is that the serum half-life of a molecule with an FcRn binding site is extended relative to an otherwise identical molecule lacking such a site.

It is desirable that the Hg-C_{H}2-C_{H}3 portion of the mimetibodies of the invention contain a FcRn binding site at the junction of the C_{H}2 and C_{H}3 regions. It is expected that such FcRn sites will increase the serum half-life of the mimetibodies of the invention as well as improve other pharmacokinetic properties relative to a melanocortin receptor binding molecule, such as alpha-MSH alone. In the mimetibodies of the invention FcRn sites may be modified or added by removal, deletion, insertion or substitution of amino acids. Typically, such modifications are used to improve the binding of a given site to the FcRn. One example of a human FcRn binding sites is the sequence MISRTPTVLHQHNHY (SEQ. ID. NO.: 69) found in both IgG₁ and IgG₄ antibodies. Other FcRn binding sites are well known by those skilled in the art.

Antibodies with different isotypes, such as IgG₄ and IgG₁, may contain glycosylation sites. Glycosylation of these sites can alter the properties and activites of antibody molecules. Antibody molecules may be N-glycosylated or O-glycosylated. N-glycosylation of antibody amino acid residue side chains containing nitrogen atoms (*e.g*., Asn) can modulate antibody Fc effector functions such as ADCC by conferring a cytolytic activity to N-glycosylated antibody molecules. This ADCC associated cytolytic activity causes the lysis of cells effected by such N-glycosylated antibodies. Alternatively, an antibody molecule may be O-glycosylated by modification of amino acid residue side chains containing oxygen atoms (*e.g*., Ser or Thr).

O-glycosylation can decrease the serum half-life of an antibody molecule through increased lectin mediated clearance of O-glycosylated antibody molecules from the serum. Additionally, O-glycosylation can cause undesirable increases in antibody heterogeneity due to differing extents of O-glycosylation between various antibody molecules. Lastly, both O-glycosylation and N-glycosylation can alter the structure dependent properties of antibody molecules such as binding affinity and immunogenicity.

Like the antibody molecules they mimic, the mimetibody polypeptides of the invention may also be post-translationally modified by N-glycosylation and O-glycosylation. In most instances, it is desirable to limit the N-glycosylation of the mimetibodies of the invention to minimize cytolytic activity. N-glycosylation can be limited by the removal or substitution of amino acid residues, such as Asn, which are typically N-glycosylated. It is also desirable to limit mimetibody O-glycosylation to minimize lectin-mediated clearance, mimetibody heterogeneity and the alteration of structure dependent mimetibody properties such as binding affinity and immunogenicity. One way to minimize O-linked glycosylation in the mimetibodies of the invention is to substitute Ala residues for Thr residues in the V2 portion of the polypeptides of the invention.

The V2 amino acid sequence TLVAVSS (SEQ ID NO: 34) is exemplary of such a Thr to Ala substitution; this particular V2 substitution can also be obtained by a Thr to Ala substitution at postion 47 of SEQ ID NO: 62. Those skilled in the art also will recognize other ways to control N-linked and O-linked glycosylation including modulation of glycosylase enzyme activity.

The monomeric structure Mp-Lk-V2-Hg-C_{H}2-C_{H}3 of the mimetibody polypeptides of the invention can be linked to "t" other monomers where t is an integer from 1 to 10. Such linking can occur through non-covalent interactions or covalent linkages such as a Cys-Cys disulfide bond. In this way multimeric structures such as dimers and higher order multimers of the polypeptides of the invention can be formed. It is expected that dimerization of the polypeptides of the invention will increase the affinity of these polypeptides to melanocortin receptors such as MC4R. The term "multimers" as used herein means molecules that have quaternary structure and are formed by the association of two or more subunits.

The polypeptides of the invention can optionally comprise at the amino terminus, a amino terminal portion of an immunoglobulin variable region, designated V1 as shown in Formula II:

(V1-Mp-Lk-V2-Hg-C_{H}2-C_{H}3) ₍ₜ₎ (II)

Exemplary V1 amino acid sequences include QIQ and QVQ.

The polypeptides of the invention may also comprise secretory signals necessary to facilitate protein secretion or other signals necessary for protein trafficking in the cell. An exemplary secretory signal sequence is MAWVWTLLFLMAAAQSIQA (SEQ ID NO: 69). Those skilled in the art will recognize other secretory signals.

In one embodiment the polypeptides of the invention comprise SEQ ID NO: 60 or 62. SEQ ID NO: 62 represents a (V1-Mp-Lk-V2-Hg-C_{H}2-C_{H}3)₍ₜ₎ melanocortin receptor binding alpha-MSH polypetide of generic formula (II) which has the secretory signal MAWVWTLLFLMAAAQSIQA (SEQ ID NO: 69) fused to its amino terminus. SEQ ID NO: 60 represents a (Mp-Lk-V2-Hg-C_{H}2-C_{H}3) ₍ₜ₎ melanocortin receptor binding alpha-MSH polypetide of generic formula (I). No secretory signal is present in SEQ ID NO: 60.

Another aspect of the present invention is a polynucleotide comprising, complementary to or having significant identity with, a polynucleotide encoding at least one melanocortin receptor binding mimetibody. Other aspects of the present invention include vectors comprising at least one polynucleotide molecule encoding a melanocortin receptor binding mimetibody. In a different aspect the invention provides a cell comprising a vector of the invention or a cell expressing a mimetibody polypeptide of the invention. The polynucleotides, vectors and cells may be used to produce the mimetibody polypeptides of the invention.

In one embodiment, the polynucleotides of the invention comprise SEQ ID NO: 59 or SEQ ID NO: 61 or a polynucleotide complementary to SEQ ID NO: 59 or SEQ ID NO: 61. SEQ ID NO: 59 is a cDNA encoding a (Mp-Lk-V2-Hg-C_{H}2-C_{H}3) ₍ₜ₎ melanocortin receptor binding alpha-MSH polypetide of generic formula (I) which lacks a signal sequence. SEQ ID NO: 61 is a cDNA encoding a (V1-Mp-Lk-V2-Hg-C_{H}2-C_{H}3) ₍ₜ₎ melanocortin receptor binding alpha-MSH polypetide of generic formula (II) which has a secretory signal fused to its amino terminus .

In one embodiment, the polynucleotides of the invention comprise a polynucleotide encoding the polypeptide of SEQ ID NO: 60 or SEQ ID NO: 62. Exemplary nucleic acid sequences that encode the polypeptide sequences shown in SEQ ID No 60 or SEQ ID NO: 62 are shown in SEQ ID NO 59 or SEQ ID NO: 61, respectively. Also provided are polynucleotides that are substantially identical to the above described polynucleotides.

The term "substantially identical" in the context of polynucleotides means that a given polynucleotide sequence is identical to a polynucleotide sequence of the invention, or portion thereof, in at least 60% or at least about 70% or at least about 80% or at least about 90% or at least about 95-98% of the nucleotides. Percent identity between two polynucleotide sequences can be determined by pairwise alignment using the default settings of the AlignX module of Vector NTI v.9.0.0 (Invitrogen Corp., Carlsbad, CA).

Typically, the polynucleotides of the invention are used in expression vectors for the preparation of the mimetibody polypeptides of the invention. Vectors within the scope of the invention provide necessary elements for eukaryotic expression and include viral promoter driven vectors, such as CMV promoter driven vectors, e.g., pcDNA3.1, pCEP4, and their derivatives, Baculovirus expression vectors, Drosophila expression vectors, and expression vectors that are driven by mammalian gene promoters, such as human Ig gene promoters. Other examples include prokaryotic expression vectors, such as T7 promoter driven vectors, e.g. pET41, lactose promoter driven vectors and arabinose gene promoter driven vectors.

The present invention also relates to a cell that expresses a mimetibody of the invention or comprises a vector of the invention. Such a cell can be prokaryotic or eukaryotic. Exemplary eukaryotic cells are mammalian cells, such as but not limited to, COS-1, COS-7, HEK293, BHK21, CHO, BSC-1, HepG2, 653 , SP2/0, NS0 293, HeLa, myeloma, lymphoma cells or any derivative thereof. Most preferably, the eukaryotic cell is a HEK293, NS0 SP2/0, or CHO cell. *E. coli* is an exempary prokaryotic cell. A cell according to the invention may be generated by transfection, cell fusion, immortalization, or other procedures that are well known in the art. Polynucleotides transfected into a cell may be extrachromasomal or stably integrated into the chromosome of the cell.

The mimetibodies of the invention can be made more compatible with a given host cell by modification of the Hg-C_{H}2-C_{H}3 portion of the polypeptide. For example, when a mimetibody of the invention is expressed recombinantly in a bacterial cell such as E. *coli,* the Pro-Ala sequence in the Hg element may be removed to prevent digestion by the *E. coli* enzyme proline iminopeptidase. Similarly, a portion of the Hg element can be deleted or substituted with other amino acids in the mimetibodies of the invention to prevent heterogeneity in the products expressed in a selected host cell.

The present invention further provides a method to produce a mimetibody polypeptide comprising the steps of culturing a cell of the invention and purifying an expressed mimetibody polypeptide of the invention. Cell components, such as those necessary for in vitro transcription and translation, may also be used to express the polypeptides of the invention. The present invention encompasses mimetibodies produced by both methods. Expressed mimetibody polypeptides can be recovered and purified from cells or cell component based systems by methods well known in the art including, but not limited to, protein A purification, ammonium sulfate or ethanol precipitation, acid extraction, anion or cation exchange chromatography, phosphocellulose chromatography, hydrophobic interaction chromatography, affinity chromatography, hydroxylatpatite chromatography and lectin chromatography. High performance liquid chroatography (HPLC) can also be employed for purification. Typically purfication will require a combination of several different methods.

Another aspect of the present invention is a pharmaceutical composition comprising an effective amount of at least one mimetibody polypeptide and a pharmaceutically acceptable carrier or diluent. The term "effective amount" generally refers to the quantity of mimetibody necessary for effective therapy, *i.e*., the partial or complete alleviation of the symptom or disorder for which treatment was sought. The composition can optionally comprise at least one further compound, protein or composition useful for treating obesity and the other conditions described below. The parmaceutically acceptable carrier or diluent in the compositions can be a solution, suspension, emulsion, colloid or powder. Those skilled in the art will recognize other parmaceutically acceptable carriers and diluents.

Another aspect of the present invention is a polypeptide of the invention for use in medicine. The polypeptide may be for use in a method of modifying the biological activity of a melanocortin receptor in a cell, tissue or organ comprising contacting the pharmaceutical compositions of the invention with the cell, tissue or organ. The polypeptide of the invention may be for use in a method to modify melanocortin receptor activity in the brain, brain tissue, or brain cells. Alternatively, the polypeptide of the invention may be for use in a method to modify melanocortin receptor activity in other peripheral cells or tissues such as muscle, or other organs such as the stomach. Those skilled in the art will recognize other cells, tissues or organs, which may be used.

Another aspect of the invention is a polypeptide of the invention for modulating at least one melanocortin receptor-mediated condition comprising administering a pharmaceutical composition of the invention to a patient in need thereof. The pharmaceutical compositions of the invention can be administered by any suitable route. Such routes may be intrathecal, intranasal, peripheral (*e.g*., subcutaneous, intramuscular, intradermal, intravenous) or by any other means known in the art. As described previously, abnormal melanocortin receptor activity has been implicated in a number of pathological conditions, such as obesity and Type 2 diabetes. The mimetibody polypeptides of the invention may be also be used to modulate other melanocortin receptor mediated conditions such as male and female erectile dysfunction, inflammation, congestive heart failure, central nervous system disorders, nerve damage, infectious disease, pulmonary disease, skin disease, fever and pain.

The present invention is further described with reference to the following examples. These examples are merely to illustrate aspects of the present invention and are not intended as limitations of this invention.

### Example 1

### Alpha-MSH Mimetibody and Expression Vector Construction

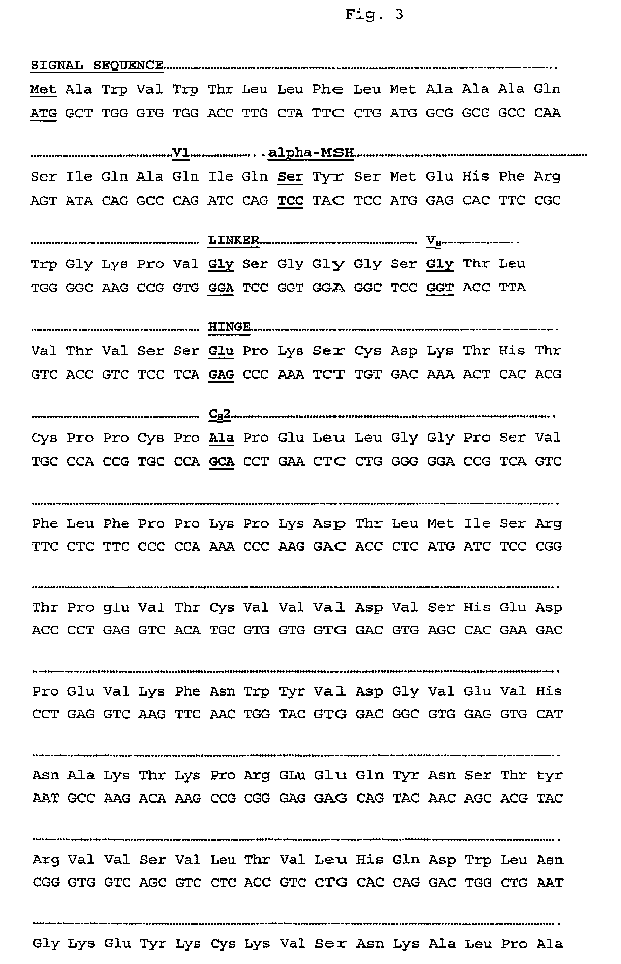

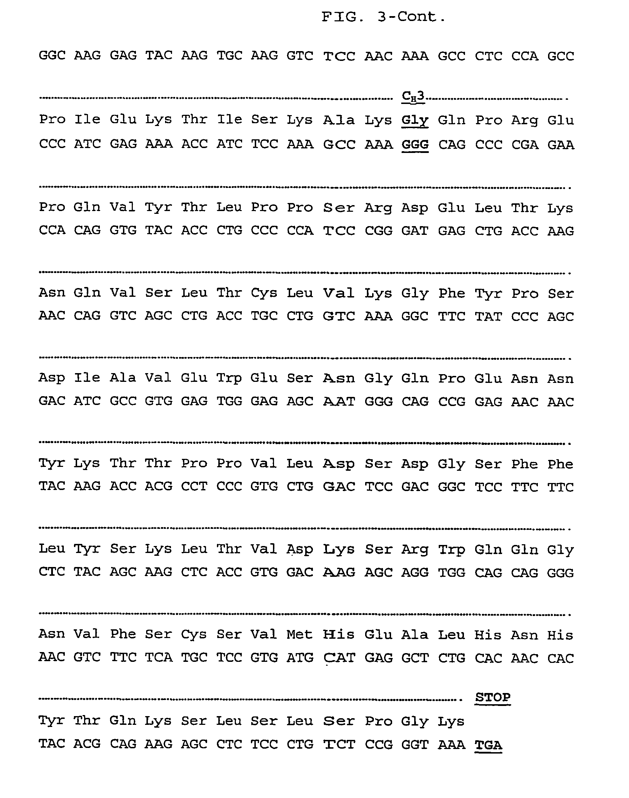

An alpha-MSH mimetibody protein comprising a secretory signal sequence, an alpha-MSH peptide sequence, a linker sequence, V_{H} sequence, a hinge sequence, a human IgG₁ C_{H}2 sequence and a human IgG₁ C_{H}3 sequence was designed (Fig. 3 and SEQ ID NO. 62) Analytical data, *e.g.,* mass spectroscopy, has confirmed that a mature polypeptide is generated (61,344.6 for G1/G1 form). Nucleic acid sequences encoding this alpha-MSH mimetibody protein (Fig. 3; SEQ ID NO: 61) were generated using standard molecular biology techniques. Nucleic acid sequences encoding the alpha-MSH mimetibody sequence were subcloned into the p2389 expression vector to generate an alpha-MSH mimetibody expression vector (SEQ ID NO: 63).

### Example 2

### Alpha-MSH Mimetibody Expression

The alpha-MSH mimetibody was transiently expressed in HEK293E cells. Cells were cultured using standard conditions and transiently transfected with the alpha-MSH mimetibody expression vector using Lipofectamine 2000 (Invitrogen, Carlsbad, CA) as directed by the manufacturer. 24 h after transfection cells were transferred to a serum free media formulation and cultured for 5 days. The culture media was then removed and centrifuged to remove debris. Clarified media was incubated with Protein A-Sepharose^{™} (HiTrap rProtein A FF, Amersham Biosciencies, Piscataway, NJ) and proteins were eluted from the Protein A-Sepharose^{™} conjugate as directed by the manufacturer. The eluted protein solution was then further purified via Superose^{™} 12 size exclusion chromatography (Superose 12 10/300 GL, Amersham Biosciencies, Piscataway, NJ) using standard methods. Column eluant was then subjected to SDS-PAGE and visualized by silver and Coomassie blue staining. Western blots were then prepared and the blots were probed with either an Fc specific primary antibody or an alpha-MSH specific primary antibody. Together, the Western Blot and SDS-PAGE staining results indicated that a purified alpha-MSH mimetibody, composed of two polypeptide chains, had been obtained from the transiently transfected HEK293 cells.

### Example 3

### Alpha-MSH Mimetibody Binds MC4R

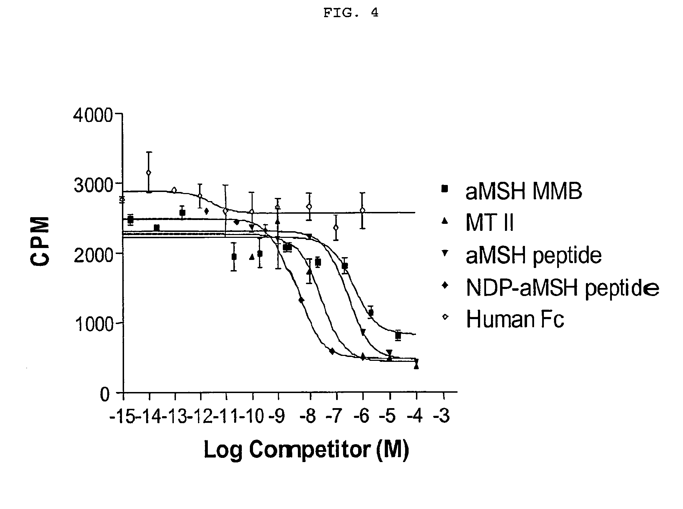

The alpha-MSH mimetibody binds to MC4R and can compete with radiolabeled [Nle(4), D-Phe(7)]-alpha-MSH (NDP-alpha-MSH) agonist molecules for MC4R binding (Fig. 4). MC4R is a receptor for alpha-MSH. alpha-MSH binding to recombinantly expressed MC4R in HEK293 cell membranes (Perkin Elmer Life and Analytical Sciences, Boston, MA) was examined by competive binding assays in which increasing amounts of unlabeled MC4R agonists (positive controls) and the Fc domain of a human antibody (negative control) were added to assay cocktails containing [¹²⁵I]-NDP-alpha-MSH as indicated in Fig. 4. The unlabeled MC4R agonists were melanotan II (MTII; an alpha MSH analog), alpha-MSH, and NDP-alpha-MSH. Alpha-MSH mimetibody binding to MC4R was stable after two weeks of storage at 4°C, -20°C, and -80°C in PBS (phosphate buffered saline) as assessed by competive binding assays.

Competivive binding assays were performed using Scintillation Proximity Assays^{®} (Amersham Biosciences Corp, Piscataway, NJ) as directed by the assay manufacturer. Assay cocktails contained [¹²⁵I]-NDP-alpha-MSH at EC80, *i.e*., ∼0.5 nM, 0.1 µg of MC4R membranes, 1 mM MgSO₄, 1.5 mM CaCl₂, 25 mM Hepes, 0.2% BSA, 1 mM 1,10-phenthroline, an assay manufacturer recommended quantity of protease inhibitor cocktail (Roche Diagnostics Corp., Indianapolis, IN) and SPA beads. Light emission from Scintillation Proximity Assay^{®} beads was measured with a Packard Top Count NXT Instrument (Perkin Elmer Life and Analytical Sciences, Boston, MA) for 5 minutes.

### Example 4

### Alpha-MSH Mimetibody Activates MC4R

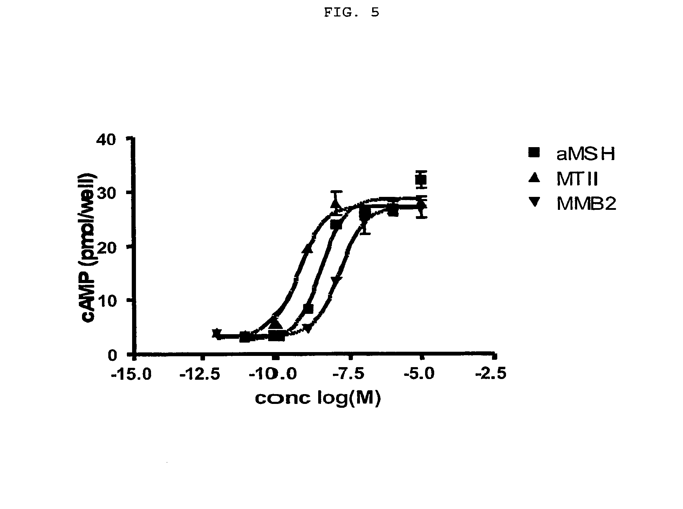

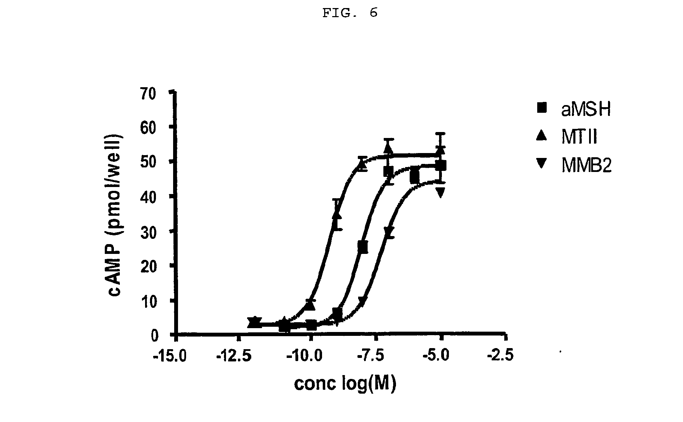

The alpha-MSH mimetibody can activate MC4R signalling to increase cAMP production in CHOK1 cells expressing MC4R (Fig. 5 and Fig. 6). MC4R is a seven transmembrane (7TM) G-protein coupled receptor. Activation of MC4R by ligand or agonist results in an increase in cyclic AMP levels (cAMP).

MC4R receptor activation assays were performed using two different clonal CHOK1 cell lines stably transfected with a MC4R expression vector and expressing MC4R. Clone 1 (Fig. 5) expressed MC4R at high levels relative to Clone 2 (Fig. 6). Clone 1 and Clone 2 cells were grown as a monolayer using standard culture conditions to a density of approximately 100,000 cells/well and then incubated with increasing amounts (0-100 µM) of alpha-MSH, MTII, or alpha-MSH mimetibody for 15 minutes as indicated in Fig. 5 and Fig. 6. Cells were then lysed and cAMP assays were performed using the cAMP-Screen Direct^{™} Chemiluminescent Immunoassay System (Applied Biosystems, Foster City, CA) as directed by the manufacturer. EC₅₀ values from cAMP assays using Clone 1 (Fig. 5) and Clone 2 (Fig. 6) are listed in Table 1 below

**Table 1**

| | Clone 1 | | | Clone 2 | | |
|---|---|---|---|---|---|---|
| alpha-MSH peptide (Positive control) | EC₅₀ | = | 3.29 nM | EC₅₀ | = | 9.46 nM |
| MT II (Positive control) | EC₅₀ | = | 0.52 nM | EC₅₀ | = | 0.52 nM |
| alpha-MSH mimetibody | EC₅₀ | = | 14.36 nM | EC₅₀ | = | 52.4 nM |

### Example 5

### Alpha-MSH Mimetibody Administration Decreases Animal Food Intake and Body Weight

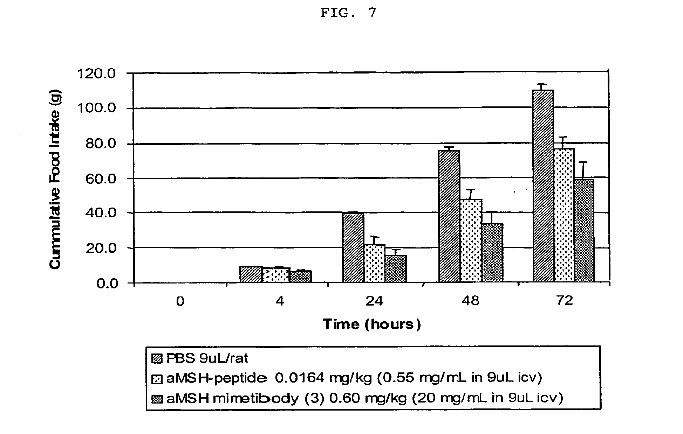

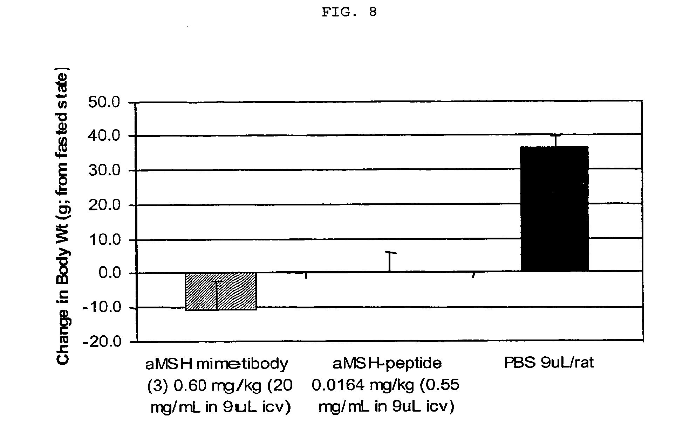

Alpha-MSH mimetibody administration to *Rattus norvegicus* brain ventricules decreases animal food intake (Fig. 7) and body weight (Fig. 8). Alpha-MSH mimetibody was supplied to brain ventricules by intracerebroventricular injections (ICV) via a cannula surgically inserted into the left lateral brain ventricle.

Cannulae were surgically inserted into male Sprague-Dawley or Wistar rats weighing 250 g to 350 g. Cannula placement coordinates were as follows: -0.8 mm from bregma, -4.5 mm ventral and -1.5 posterior-anterior. Animals recovered for 7 to 10 days after surgery. Animals were acclimatized to the experimental procedures by both daily handling and mock injection, in order to minimize stress. In addition animals were submitted to the reversal of dark-light cycle.

Proper cannula placement was confirmed by an angiotensin II test. The test confirmed proper cannula placement if the ICV administration of 10 ng of angiotensin II via the cannula caused the rats to drink 5-10 ml of water in 30 minutes. Only animals that passed this angiotensin II test were used in food intake experiments.

Animals were fasted for 18-24 hours and alpha-MSH mimetibody, alpha-MSH (positive control), or PBS (negative control) were then administered to the brain ventricles via the cannula at an injection rate of 9 µl/min. Each treatment group had a minimum of 7 animals. Treatments and dosages were as indicated in Fig. 7 and Fig. 8.

Food and water was given to the animals after injection. The amount of food and water consumed was measured at 0 h, 4 h, 24 h, 48 h and 72 h (Fig. 7) after injection. Body weight at 72 hours post injection was measured as shown in Fig. 8.

The present invention now being fully described, it will be apparent to one of ordinary skill in the art that many changes and modifications can be made thereto without departing from the scope of the appended claims.

## Claims

1. A polypeptide according to formula (I):
(Mp-Lk-v2-Hg-C_{H}2-C_{H}3) (t) (I)
where Mp is a melanocortin receptor binding molecule, Lk is a polypeptide or chemical linkage, V2 is a portion of a C-terminus of an immunoglobulin variable region, Hg is at least a portion of an immunoglobulin variable hinge region, C_{H}2 is an immunoglobulin heavy chain C_{H}2 constant region and C_{H}3 is an immunoglobulin heavy chain C_{H}3 constant region and t is independently an integer from 1 to 10.

2. The polypeptide of claim 1 wherein Mp is a biologically active fragment of SEQ ID NO: 2, 4, 6, 8, 10, 12, 14, 16, or 18; or Mp has the amino acid sequence shown in SEQ ID NO: 2, 4, 6, 8, 10, 12, 14, 16, or 18.

3. The polypeptide of claim 1 wherein the polypeptide binds to at least one melanocortin receptor.

4. The polypeptide of claim 3 wherein the melanocortin receptor is a melanocortin 4 receptor.

5. A polypeptide comprising SEQ ID NO: 60 or 62.

6. A polynucleotide encoding a polypeptide according to any one of claims 1 to 5.

7. A polynucleotide comprising SEQ ID NO: 59 or SEQ ID NO: 61 or a polynucleotide complementary to SEQ ID NO: 59 or SEQ ID NO: 61.

8. A polynucleotide comprising a polynucleotide encoding the polypeptide of SEQ ID NO: 60 or SEQ ID NO: 62.

9. A vector comprising the polynucleotide of claim 7 or 8.

10. The vector of claim 9 comprising SEQ ID NO: 63.

11. A cell expressing a polypeptide according to any one of claims 1 to 5.

12. A cell comprising the vector of claim 9.

13. The cell of claim 12 wherein the cell is a HEK293 derived cell.

14. A method to produce a polypeptide comprising the steps of culturing the cell of claim 11 and purifying the expressed polypeptide.

15. A pharmaceutical composition comprising an effective amount of at least one polypeptide according to any one of claims 1 to 5 and a pharmaceutically acceptable carrier or diluent.

16. A polypeptide according to any of claims 1 to 5 for use in medicine.

17. A polypeptide according to any of claims 1 to 5 for modulating at least one melanocortin receptor mediated condition.

18. The polypeptide of claim 17 wherein the melanocortin receptor mediated condition is obesity.

## Patentansprüche

1. Ein Polypeptid gemäß der Formel (I):
(Mp-Lk-V2-Hg-C_{H}2-C_{H}3) ₍ₜ₎ (I)
wobei Mp ein Melanocortin-Rezeptor-bindendes Molekül ist, Lk ein Polypeptid oder eine chemische Verknüpfung ist, V2 ein Teil eines C-Terminus einer variablen Region eines Immunglobulins ist, Hg mindestens ein Teil einer variablen Gelenkregion eines Immunglobulins ist, C_{H}2 eine C_{H}2 konstante Region einer schweren Immunglobulinkette ist und C_{H}3 eine C_{H}3 konstante Region einer schweren Immunglobulinkette ist und t unabhängig eine ganze Zahl von 1 bis 10 ist.

2. Das Polypeptid nach Anspruch 1, wobei Mp ein biologisch aktives Fragment von SEQ ID NO: 2, 4, 6, 8, 10, 12, 14, 16 oder 18 ist; oder Mp die in SEQ ID NO: 2, 4, 6, 8, 10, 12, 14, 16 oder 18 gezeigte Aminosäuresequenz aufweist.

3. Das Polypeptid nach Anspruch 1, wobei das Polypeptid an mindestens einen Melanocortin-Rezeptor bindet.

4. Das Polypeptid nach Anspruch 3, wobei der Melanocortin-Rezeptor ein Melanocortin-4-Rezeptor ist.

5. Ein Polypeptid, das SEQ ID NO: 60 oder 62 umfasst.

6. Ein Polynukleotid, das für ein Polypeptid nach einem der Ansprüche 1 bis 5 kodiert.

7. Ein Polynukleotid, das SEQ ID NO: 59 oder SEQ ID NO: 61 oder ein Polynukleotid, das komplementär zu SEQ ID NO: 59 oder SEQ ID NO: 61 ist, umfasst.

8. Ein Polynukleotid, das ein Polynukleotid umfasst, welches für das Polypeptid der SEQ ID NO: 60 oder SEQ ID NO: 62 kodiert.

9. Ein Vektor, der das Polynukleotid nach Anspruch 7 oder 8 umfasst.

10. Der Vektor nach Anspruch 9, der SEQ ID NO: 63 umfasst.

11. Eine Zelle, die ein Polypeptid nach einem der Ansprüche 1 bis 5 exprimiert.

12. Eine Zelle, die den Vektor nach Anspruch 9 umfasst.

13. Die Zelle nach Anspruch 12, wobei die Zelle eine von HEK293 abgeleitete Zelle ist.

14. Ein Verfahren zur Produktion eines Polypeptids, das die Schritte des Kultivierens der Zelle nach Anspruch 11 und des Aufreinigens des exprimierten Polypeptids umfasst.

15. Eine pharmazeutische Zusammensetzung, die eine wirksame Menge mindestens eines Polypeptids nach einem der Ansprüche 1 bis 5 und einen pharmazeutisch akzeptablen Träger oder ein Verdünnungsmittel umfasst.

16. Ein Polypeptid nach einem der Ansprüche 1 bis 5 zur medizinischen Verwendung.

17. Ein Polypeptid nach einem der Ansprüche 1 bis 5 zur Modulierung mindestens eines Melanocortin-Rezeptor-vermittelten Zustandes.

18. Das Polypeptid nach Anspruch 17, wobei der Melanocortin-Rezeptor-vermittelte Zustand Fettleibigkeit ist.

## Revendications

1. Polypeptide selon la formule (I):
(Mp-Lk-V2-Hg-C_{H}2C_{H}3) (t) (I)
où Mp est une molécule de liaison du récepteur de la mélanocortine, Lk est un polypeptide ou une liaison chimique, V2 est une portion d'un C-terminus d'une région variable d'immunoglobuline, Hg est au moins une portion d'une région de charnière variable d'immunoglobuline, C_{H}2 est une région constante de C_{H}2 de chaîne lourde d'immunoglobuline et C_{H}3 est une région constante de C_{H}3 de chaîne lourde d'immunoglobuline et t est indépendamment un entier de 1 à 10.

2. Polypeptide selon la revendication 1, où Mp est un fragment biologiquement actif de la SEQ ID NO: 2, 4, 6, 8, 10, 12, 14, 16 ou 18; ou Mp a la séquence des acides aminés indiquée dans la SEQ ID NO: 2, 4, 6, 8, 10, 12, 14, 16 ou 18.

3. Polypeptide selon la revendication 1, où le polypeptide se lie à au moins un récepteur de la mélanocortine.

4. Polypeptide selon la revendication 3, où le récepteur de la mélanocortine est un récepteur de la mélanocortine 4.

5. Polypeptide comprenant la SEQ ID NO: 60 ou 62.

6. Polynucléotide codant pour un polypeptide selon l'une quelconque des revendications 1 à 5.

7. Polynucléotide comprenant la SEQ ID NO: 59 ou la SEQ ID NO: 61 ou un polynucléotide complémentaire à la SEQ ID NO: 59 ou à la SEQ ID NO: 61.

8. Polynucléotide comprenant un polynucléotide codant pour le polypeptide de la SEQ ID NO: 60 ou la SEQ ID NO: 62.

9. Vecteur comprenant le polynucléotide de la revendication 7 ou 8.

10. Vecteur selon la revendication 9, comprenant la SEQ ID NO: 63.

11. Cellule exprimant un polypeptide selon l'une quelconque des revendications 1 à 5.

12. Cellule comprenant le vecteur de la revendication 9.

13. Cellule selon la revendication 12, où la cellule est une cellule dérivée de HEK293.

14. Méthode pour produire un polypeptide comprenant les étapes consistant à cultiver la cellule de la revendication 11 et à purifier le polypeptide exprimé.

15. Composition pharmaceutique comprenant une quantité efficace d'au moins un polypeptide selon l'une quelconque des revendications 1 à 5 et un support ou diluant pharmaceutiquement acceptable.

16. Polypeptide selon l'une quelconque des revendications 1 à 5 pour utilisation en médecine.

17. Polypeptide selon l'une quelconque des revendications 1 à 5 pour moduler au moins une condition régulée par le récepteur de la mélanocortine.

18. Polypeptide selon la revendication 17, où la condition régulée par le récepteur de la mélanocortine est l'obésité.
